# EUROPEAN PATENT SPECIFICATION

(11) **EP 1 784 220 B1**
(45) Date of publication and mention of the grant of the patent: **20.12.2017**
(21) Application number: 05806350.4
(22) Date of filing: 26.08.2005
(51) Int. Cl.: A61K 39/395, G01N 33/569, G01N 33/53, A61K 31/7088, G01N 33/50, C12Q 1/68, C12Q 1/18, A61P 31/04, A61K 39/085, A61K 38/16, A61K 31/7105, C07K 16/12

(54) **BACTERIAL IRON ACQUISITION TARGETS**
BAKTERIELLE EISEN-AKQUISITIONS-TARGETS
CIBLES BACTERIENNES D'ACQUISITION DE FER

(30) Priority: 26.08.2004 US 604769 P
(43) Date of publication of application: 16.05.2007
(73) Proprietor: THE UNIVERSITY OF WESTERN ONTARIO, London, Ontario N6A 5B8 (CA)
(72) Inventor: HEINRICHS, David, E., London, Ontario N5Y 2X2 (CA); DALE, Suzanne, Rochester, NY 14620 (US)
(74) Representative: Lee, Nicholas John
(86) International application number: PCT/IB2005/003576
(87) International publication number: WO 2006/021893

(56) References cited:
- WO-A1-99/47662
- WO-A2-02/094868
- DALE SUZANNE E ET AL: "Role of siderophore biosynthesis in virulence of Staphylococcus aureus: identification and characterization of genes involved in production of a siderophore." INFECTION AND IMMUNITY JAN 2004, vol. 72, no. 1, January 2004 (2004-01), pages 29-37, XP002478683 ISSN: 0019-9567
- CABRERA G ET AL: "Molecular characterization of the iron-hydroxamate uptake system in Staphylococcus aureus." APPLIED AND ENVIRONMENTAL MICROBIOLOGY FEB 2001, vol. 67, no. 2, February 2001 (2001-02), pages 1001-1003, XP002478684 ISSN: 0099-2240
- DALE SUZANNE E ET AL: "Involvement of SirABC in iron-siderophore import in Staphylococcus aureus." JOURNAL OF BACTERIOLOGY DEC 2004, vol. 186, no. 24, December 2004 (2004-12), pages 8356-8362, XP002478685 ISSN: 0021-9193
- SEBULSKY M T ET AL: "Identification and characterization of a membrane permease involved in iron-hydroxamate transport in Staphylococcus aureus." JOURNAL OF BACTERIOLOGY AUG 2000, vol. 182, no. 16, August 2000 (2000-08), pages 4394-4400, XP002478686 ISSN: 0021-9193
- HEINRICHS J H ET AL: "Identification and characterization of SirA, an iron-regulated protein from Staphylococcus aureus." JOURNAL OF BACTERIOLOGY MAR 1999, vol. 181, no. 5, March 1999 (1999-03), pages 1436-1443, XP002478687 ISSN: 0021-9193
- SPEZIALI CRAIG D ET AL: "Requirement of Staphylococcus aureus ATP-binding cassette-ATPase FhuC for iron-restricted growth and evidence that it functions with more than one iron transporter." JOURNAL OF BACTERIOLOGY MAR 2006, vol. 188, no. 6, March 2006 (2006-03), pages 2048-2055, XP002478688 ISSN: 0021-9193
- HEINRICHS ET AL: 'Identification and characterization of SirA, an iron-regulated protein from Staphylococcus aureus' J. BACTERIOLOGY vol. 181, no. 5, March 1999, pages 1426 - 1443, XP002478687
- SEBULSKY ET AL: 'Identification and characterization of a membrane permease involved in iron-hydroxamate transport in Staphylococcus aureus' J. BACTERIOLOGY vol. 182, no. 16, August 2000, pages 4394 - 4400, XP002478686
- DALE ETAL: 'Involvement of SirABC in iron-siderophore import in Staphylococcus aureus' J. BACTEIOLOGY vol. 186, no. 24, December 2004, pages 8356 - 8362, XP002478685

## Description

### Background

*Staphylococcus aureus* (*S. aureus*) is a prevalent human pathogen that causes a wide range of infections ranging from minor skin lesions, impetigo and food poisoning to more serious diseases such as sepsis, endocarditis, osteomyelitis, pneumonia, bacteremia, and toxic shock syndrome (Archer (1998) Clin. Infect. Dis. 26:1179-1181). Initially, penicillin could be used to treat even the worst *S. aureus* infections. However, the emergence of penicillin-resistant strains of *S. aureus* has reduced the effectiveness of penicillin in treating *S. aureus* infections and most strains of *S. aureus* encountered in hospital infections today do not respond to penicillin. Penicillin-resistant strains of *S. aureus* produce a lactamase, which converts penicillin to pencillinoic acid, and thereby destroys antibiotic activity. Furthermore, the lactamase gene often is propagated episomally, typically on a plasmid, and often is only one of several genes on an episomal element that, together, confer multidrug resistance.

Methicillins, introduced in the 1960s, largely overcame the problem of penicillin resistance in *S. aureus.* These compounds conserve the portions of penicillin responsible for antibiotic activity and modify or alter other portions that make penicillin a good substrate for inactivating lactamases. However, methicillin resistance has emerged in *S. aureus,* along with resistance to many other antibiotics effective against this organism, including vancomycin, aminoglycosides, tetracycline, chloramphenicol, macrolides and lincosamides. In fact, methicillin-resistant strains of *S. aureus* generally are multiply drug resistant. Methicillian-resistant *S. aureus* (MRSA) has become one of the most important nosocomial pathogens worldwide and poses serious infection control problems. Drug resistance of *S. aureus* infections poses significant treatment difficulties; which are likely to get much worse unless new therapeutic agents are developed. There is thus an urgent unmet medical need for new and effective therapeutic agents to treat *S. aureus* infections.

WO 02/094868 generally discloses amino acid and nucleotide sequences of *Staphylococcus aureus* proteins including FhuC. The document suggests that compositions comprising proteins, antibodies, and/or nucleic acids disclosed therein may be suitable as immunogenic compositions.

WO 99/47662 discloses eleven *S. aureus* genes and the polypeptides they encode including cbrA, cbrB and cbrC. Also provided are vectors, host cells, antibodies and recombinant methods for producing the same. The disclosure further relates to screening methods for identifying agonists and antagonists of *S. aureus* polypeptide activity. The disclosure additionally relates to diagnostic methods for detecting *Staphylococcus* nucleic acids, polypeptides and antibodies in a biological sample and vaccines for the prevention or attenuation of infection by *Staphylococcus.*

### Summary

In one aspect, the present invention provides a method for identifying an agent that inhibits an interaction between *Staphylococcus aureus (S. aureus)* proteins selected from the group consisting of SirA and SirB, SirA and SirC, SirB and SirC, SirB and FhuC and SirC and FhuC comprising:
(i) contacting the proteins, or nucleic acid encoding the proteins, in the presence and absence of an agent under conditions permitting the interaction between the two proteins; and
(ii) determining the level of interaction between the proteins, wherein a different level of interaction between the proteins in the presence of the agent relative to in the absence of the agent indicates that the agent inhibits the interaction between the proteins.

In an embodiment, the agent is a small organic molecule, a peptide, a polypeptide, a peptide mimetic or an antibody. In an embodiment, the agent is a polynucleotide, such as an antisense polynucleotide. In an embodiment, the agent is siRNA. The agent may inhibit the interaction between FhuC and SirB or SirC.

In another aspect, the invention provides the use of an agent to inhibit growth of *S. aureus* cells, wherein the agent specifically hybridizes or binds with mRNA or genomic DNA encoding a polypeptide having an amino acid sequence of SEQ ID NO: 16.

Also disclosed are novel antibiotics, including antibodies, antisense RNAs, and siRNAs that inhibit iron uptake in *Staphylococcus aureus* (*S. aureus*)*.* Screening assays for identifying agents that inhibit iron uptake in *S. aureus* are disclosed. The assay may identify agents that inhibit the interaction between SirA, SirB, SirC, staphylobactin, and/or FhuC.

The assay may identify agents that inhibit the expression of Sir polypeptides and/or nucleic acids in *S. aureus.* The assay may be a phenotypic assay that scores the growth of *S. aureus* in iron-replete media in the presence of the test compound to the absence of the test compound.

Further features and advantages of the instant disclosed inventions will now be discussed in conjunction with the following Detailed Description and Claims.

### Brief Description of the Drawings

Figure 1 shows the genetic organization of the *sbn-sirABC* locus. The three open reading frames of the *sir* operon as well as the first gene of the *sbn* operon (*sbnA*) are indicated. The positions of the insertion sites used to disrupt the *sirA* and *sirB* coding regions, generating strains H803 and H804, respectively, in the *S. aureus* Newman background are shown. Plasmids pSED43 and pSED44, used for complementation of *sirB*::tet and *sirA*::Km mutations, respectively, are shown.
Figure 2 is an immunoblot showing iron- and Fur-regulated expression of SirA in *S. aureus* Newman and its *fur::Km* derivative. Cells were grown in either iron rich (TSB, TMS + Fe) or iron-restricted (TMS, TMS + Dip) media, normalized by optical density and lysed. SirA was detected in cell lysates with rabbit polyclonal antisera directed at SirA.
Figure 3 are graphs comparing the growth of *S. aureus* Newman versus a *sirA*::Km mutant derivative (A) or a *sirB*::*Tet* mutant derivative (B) in TMS broth containing 250 µM 2,2'-dipyridyl and 50 µM FeCl₃ (inset) or 250 µM 2,2'-dipyridyl. ■, Newman; □, H803 (*sirA*::Km); ▲, Newman carrying pAW8 vector; Δ, H803 carrying pAW8; ◆, H803 carrying pSED44 grown without IPTG; 0, H803 carrying pSED44 grown with 1 mM IPTG; ○, H804 (*sirB*::Tet)*;* ▼, H804 carrying pSED43. Data are representative of three experiments.

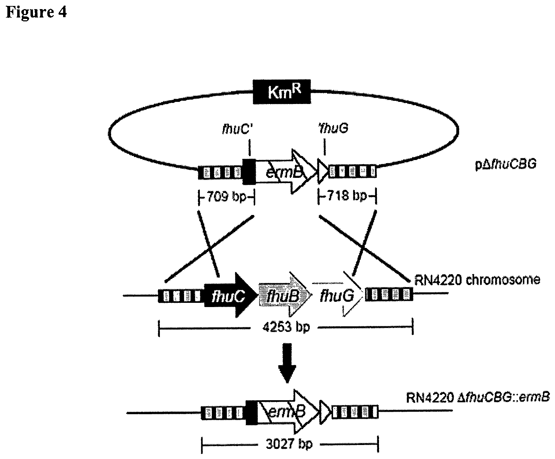

Figure 4 is a schematic diagram showing the allelic replacement of *fhuCBG* in the genome of *S. aureus* RN6390. The flanking regions of *fhuCBG, fhuC*' and '*fhuG*, were amplified from the RN6390 chromosome, ligated to either side of *ermB*, and cloned into the temperature-sensitive shuttle vector pAUL-A-Km. This construct allowed for the replacement of *fhuCBG* by *ermB* in the RN4220 genome by homologous recombination. Phage transduction was used to mobilize the mutation into the RN6390 and Newman backgrounds to yield strains H1071 and H1074, respectively. Sizes of relevant DNA fragments are indicated.

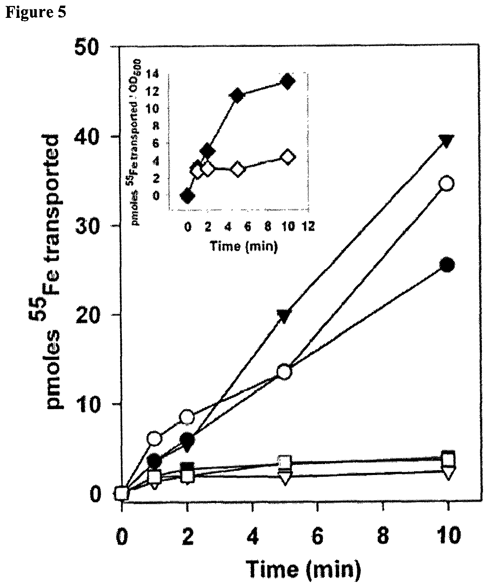

Figure 5 is a graph showing ⁵⁵Fe-staphylobactin-mediated iron transport by *S. aureus* RN6390 and H1071 derivatives grown in TMS containing 50 µM 2,2'dipyridyl. ●, RN6390; O, H1071 + pFhuC; ▼, H1071 + pFhuCBG. RN6390 (∇), H1071 + pFhuC (■) and H1071 + pFhuCBG (□) were all treated with 20 mM KCN 15 minutes prior to assay. Inset: Strains (◆, RN6390; ◊, H1071) grown prior to assay in TMS without 2,2'-dipyridyl, which was performed because strain H1071 without complementing plasmids does not grow well in the presence of 2,2'-dipyridyl, however, RN6390 is not as iron-starved in this experiment as when it is grown in the presence of 50 µM 2,2'-dipyridyl. Each point represents the pmoles of ⁵⁵Fe transported by 2 x 10⁸ cells from the assay mixture.
Figure 6 shows the nucleic acid sequence of the SirABC operon corresponding to GenBank accession number AF079518 (SEQ ID NO: 1) and the reverse complement therof (SEQ ID NO: 2).
Figure 7 shows (A) the nucleic acid (SEQ ID NO: 3), (B) the reverse complement of SEQ ID NO: 3 (SEQ ID NO: 4), and (C) the amino acid sequence of SirA (SEQ ID NO: 5).
Figure 8 shows (A) the nucleic acid (SEQ ID NO: 6), (B) the reverse complement of SEQ ID NO: 6 (SEQ ID NO: 7), and (C) the amino acid sequence of SirB (SEQ ID NO: 8).
Figure 9 shows (A) the nucleic acid (SEQ ID NO: 9), (B) the reverse complement of SEQ ID NO: 9 (SEQ ID NO: 10), and (C) the amino acid sequence of SirC (SEQ ID NO: 11).
Figure 10 shows nucleic acid of the FhuCBG operon corresponding to GenBank accession number AF251216 (SEQ ID NO: 12) and the reverse complement thereof (SEQ ID NO: 13).
Figure 11 shows (A) the nucleic acid (SEQ ID NO: 14), (B) the reverse complement of SEQ ID NO: 14 (SEQ ID NO: 15), and (C) the amino acid sequence of FhuC (SEQ ID NO: 16).

### Detailed Description

### 1. General

The present invention is based at least in part on the discovery of the role of the *Staphylococcus aureus* (*S. aureus*) *sirABC* complex in the transport of the iron-siderophore, staphylobactin, as well as the identification of *fhuC*, as encoding an ATPase required for staphylobactin uptake via the SirABC transporter. Described herein are novel antibiotics that inhibit iron uptake in *S. aureus* and methods for screening compounds to identify additional inhibitors of the SirABC iron-siderophore transport system.

### 2. Definitions

For convenience, the meaning of certain terms and phrases employed in the specification, examples, and appended claims are provided below. Unless defined otherwise, all technical and scientific terms used herein have the same meaning as commonly understood by one of ordinary skill in the art to which this invention belongs.

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. Agents may be identified by screening assays described herein below. Such agents may be inhibitors or antagonists of SirABC mediated iron transport in *Staphylococcus aureus.* The activity of such agents may render it suitable as a "therapeutic agent" which is a biologically, physiologically, or pharmacologically active substance (or substances) that acts locally or systemically in a subject.

The terms "antagonist" or "inhibitor" refer to an agent that reduces or inhibits at least one bioactivity of a protein. An antagonist may be a compound which reduces or inhibits the interaction between a protein and another: molecule, *e.g*., a target peptide or enzyme substrate. An antagonist may also be a compound that reduces or inhibits expression of a gene or which reduces or inhibits the amount of expressed protein present.

As used herein the term "antibody" refers to an immunoglobulin and any antigen-binding portion of an immunoglobulin (*e.g,* IgG, IgD, IgA, IgM and IgE) *i.e.*, a polypeptide that contains an antigen binding site, which specifically binds ("immunoreacts with") an antigen. Antibodies can comprise at least one heavy (H) chain and at least one light (L) chain inter-connected by at least one disulfide bond. The term "V_{H}" refers to a heavy chain variable region of an antibody. The term "V_{L}" refers to a light chain variable region of an antibody. In exemplary embodiments, the term "antibody" specifically covers monoclonal and polyclonal antibodies. A "polyclonal antibody" refers to an antibody which has been derived from the sera of animals immunized with an antigen or antigens. A "monoclonal antibody" refers to an antibody produced by a single clone of hybridoma cells. Techniques for generating monoclonal antibodies include, but are not limited to, the hybridoma technique (see Kohler & Milstein (1975) Nature 256:495-497); the trioma technique; the human B-cell hybridoma technique (see Kozbor et al. (1983) Immunol. Today 4:72), the EBV hybridoma technique (see Cole et al., 1985 In: Monoclonal Antibodies and Cancer Therapy, Alan R. Liss, Inc., pp. 77-96) and phage display.

Polyclonal or monoclonal antibodies can be further manipulated or modified to generate chimeric or humanized antibodies. "Chimeric antibodies" are encoded by immunoglobulin genes that have been genetically engineered so that the light and heavy chain genes are composed of immunoglobulin gene segments belonging to different species. For example, substantial portions of the variable (V) segments of the genes from a mouse monoclonal antibody, e.g., obtained as described herein, may be joined to substantial portions of human constant (C) segments. Such a chimeric antibody is likely to be less antigenic to a human than a mouse monoclonal antibody.

As used herein, the term "humanized antibody" (HuAb) refers to a chimeric antibody with a framework region substantially identical (*i.e*., at least 85%) to a human framework, having CDRs from a non-human antibody, and in which any constant region has at least about 85-90%, and preferably about 95% polypeptide sequence identity to a human immunoglobulin constant region. See, for example, PCT Publication WO 90/07861 and European Patent No. 0451216. All parts of such a HuAb, except possibly the CDRs, are substantially identical to corresponding parts of one or more native human immunoglobulin sequences. The term "framework region" as used herein, refers to those portions of immunoglobulin light and heavy chain variable regions that are relatively conserved *(i.e*., other than the CDRs) among different immunoglobulins in a single species, as defined by Kabat et al. (1987) Sequences of Proteins of Immunologic Interest, 4th Ed., US Dept. Health and Human Services. Human constant region DNA sequences can be isolated in accordance with well known procedures from a variety of human cells, but preferably from immortalized B cells. The variable regions or CDRs for producing humanized antibodies may be derived from monoclonal antibodies capable of binding to the antigen, and will be produced in any convenient mammalian source, including mice, rats, rabbits, or other vertebrates.

The term "antibody" also encompasses antibody fragments. Examples of antibody fragments include Fab, Fab', Fab'-SH, F(ab')₂, and Fv fragments; diabodies and any antibody fragment that has a primary structure consisting of one uninterrupted sequence of contiguous amino acid residues, including without limitation: single-chain Fv (scFv) molecules, single chain polypeptides containing only one light chain variable domain, or a fragment thereof that contains the three CDRs of the light chain variable domain, without an associated heavy chain moiety and (3) single chain polypeptides containing only one heavy chain variable region, or a fragment thereof containing the three CDRs of the heavy chain variable region, without an associated light chain moiety; and multispecific or multivalent structures formed from antibody fragments. In an antibody fragment comprising one or more heavy chains, the heavy chain(s) can contain any constant domain sequence (*e.g,* CHl in the IgG isotype) found in a non-Fc region of an intact antibody, and/or can contain any hinge region sequence found in an intact antibody, and/or can contain a leucine zipper sequence fused to or situated in the hinge region sequence or the constant domain sequence of the heavy chain(s). Suitable leucine zipper sequences include the jun and fos leucine zippers taught by Kostelney et al., (1992) J. Immunol., 148: 1547-1553 and the GCN4 leucine zipper described in U.S. Patent No. 6,468,532. Fab and F(ab')₂ fragments lack the Fc fragment of intact antibody and are typically produced by proteolytic cleavage, using enzymes such as papain (to produce Fab fragments) or pepsin (to produce F(ab')₂ fragments).

An antibody "specifically binds" to an antigen or an epitope of an antigen if the antibody binds preferably to the antigen over most other antigens. For example, the antibody may have less than about 50%, 20%, 10%, 5%, 1% or 0.1% cross-reactivity toward one or more other epitopes.

An "effective amount" is an amount sufficient to produce a beneficial or desired clinical result upon treatment. An effective amount can be administered to a patient in one or more doses. In terms of treatment, an effective amount is an amount that is sufficient to decrease an infection in a patient. Several factors are typically taken into account when determining an appropriate dosage to achieve an effective amount. These factors include age, sex and weight of the patient, the condition being treated, the severity of the condition and the form and effective concentration of the agent administered.

"Equivalent" when used to describe nucleic acids or nucleotide sequences refers to nucleotide sequences encoding functionally equivalent polypeptides. Equivalent nucleotide sequences will include sequences that differ by one or more nucleotide substitution, addition or deletion, such as an allelic variant; and will, therefore, include sequences that differ due to the degeneracy of the genetic code. For example, nucleic acid variants may include those produced by nucleotide substitutions, deletions, or additions. The substitutions, deletions, or additions may involve one or more nucleotides. The variants may be altered in coding regions, non-coding regions, or both. Alterations in the coding regions may produce conservative or non-conservative amino acid substitutions, deletions or additions.

As used herein, the term "ferric hydroxamate uptake system" or "*fhu* system" refers to a group of genes that encode an ABC transporter. The fhu system is encoded by five genes. *FhuC*, *fhuB*, and *fhu G* are present in an operon (*fhuCBG operon*) and encode components of an ATP-binding cassette (ABC) transporter. *FhuD1* and *fhuD2* are separately encoded and encode lipoproteins that bind ferric hydroxamate complexes with high affinity. Exemplary nucleotide and amino acid sequences for the fhuCBG operon may be found in GenBank, Accession Nos. AF251216, AAF98153, AAF98154, and AAF98155; for fhuDl, Accession No. AF325854 and AAK92085; and for fhuD2 AF325855 and AAK92086. The terms "FhuC", "FhuB", "FhuG", "FhuD1", and "FhuD2" encompass fragments or portions thereof and biologically active fragments or portions thereof.

"Homology" or alternatively "identity" refers to sequence similarity between two peptides or between two nucleic acid molecules. Homology may be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. The term "percent identical" refers to sequence identity between two amino acid sequences or between two nucleotide sequences. Identity may be determined by comparing a position in each sequence which may be aligned for purposes of comparison. When an equivalent position in the compared sequences is occupied by the same base or amino acid, then the molecules are identical at that position; when the equivalent site is occupied by the same or a similar amino acid residue (*e.g*., similar in steric and/or electronic nature), then the molecules may be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and may be used with, e.g., default settings. ENTREZ is available through the National Center for Biotechnology Information, National Library of Medicine, National Institutes of Health, Bethesda, Md. In one embodiment, the percent identity of two sequences may be determined by the GCG program with a gap weight of 1, e.g., each amino acid gap is weighted as if it were a single amino acid or nucleotide mismatch between the two sequences. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method may be utilized to align sequences. An alternative search strategy uses MPSRCH software, which runs on a MASPAR computer. MPSRCH uses a Smith-Waterman algorithm to score sequences on a massively parallel computer. This approach improves the ability to pick up distantly related matches, and is especially tolerant of small gaps and nucleotide sequence errors. Nucleic acid-encoded amino acid sequences may be used to search both protein and DNA databases. Databases with individual sequences are described in Methods in Enzymology, ed. Doolittle, supra. Databases include Genbank, EMBL, and DNA Database of Japan (DDBJ).

As used herein, the term "infection" refers to an invasion and the multiplication of microorganisms such as *S. aureus* in body tissues, which may be clinically unapparent or result in local cellular injury due to competitive metabolism, toxins, intracellular replication or antigen antibody response. The infection may remain localized, subclinical and temporary if the body's defensive mechanisms are effective. A local infection may persist and spread by extension to become an acute, subacute or chronic clinical infection or disease state. A local infection may also become systemic when the microorganisms gain access to the lymphatic or vascular system. An infection of *S. aureus* may result in a disease or condition, including but not limited to a furuncle, chronic furunculosis, impetigo, acute osteomyelitis, pneumonia, endocarditis, scalded skin syndrome, toxic shock syndrome, and food poisoning.

The term "inhibit" refers to any decrease, reduction or complete inhibition of biological activity, nucleic acid expression, or protein expression.

"Label" and "detectable label" refer to a molecule capable of detection including, but not limited to radioactive isotopes, fluorophores, chemiluminescent moieties, enzymes, enzyme substrates, enzyme cofactors, enzyme inhibitors, dyes, metal ions, ligands (*e.g*., biotin or haptens) and the like. "Fluorophore" refers to a substance or a portion thereof which is capable of exhibiting fluorescence in the detectable range. Particular examples of labels which may be used under the invention include fluorescein, rhodamine, dansyl, umbelliferone, Texas red, luminol, NADPH, alpha- or beta-galactosidase and horseradish peroxidase.

As used herein with respect to genes, the term "mutant" refers to a gene which encodes a mutant protein. As used herein with respect to proteins, the term "mutant" means a protein which does not perform its usual or normal physiological role. *S. aureus* polypeptide mutants may be produced by amino acid substitutions, deletions or additions. The substitutions, deletions, or additions may involve one or more residues. Especially preferred among these are substitutions, additions and deletions which alter the properties and activities of a *S. aureus* protein of the present invention.

The terms "polynucleotide" and "nucleic acid" are used interchangeably. They refer to a polymeric form of nucleotides of any length, either deoxyribonucleotides or ribonucleotides, or analogs thereof. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The term "recombinant" polynucleotide means a polynucleotide of genomic, cDNA, semisynthetic, or synthetic origin which either does not occur in nature or is linked to another polynucleotide in a nonnatural arrangement. An "oligonucleotide" refers to a single stranded polynucleotide having less than about 100 nucleotides, less than about, *e.g.,* 75, 50, 25, or 10 nucleotides.

The terms "polypeptide", "peptide" and "protein" (if single chain) are used interchangeably herein to refer to polymers of amino acids. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" refers to either natural and/or unnatural or synthetic amino acids, including glycine and both the D or L optical isomers, and amino acid analogs and peptidomimetics.

As used herein, the term "*sirABC* operon" refers to a group of bacterial genes comprising *sirA*, *sirB*, and *sirC* that share a common promoter. This operon has been found to be important to the iron-restricted growth of *S. aureus.* Exemplary nucleotide and amino acid sequences of *sirABC* operon may be found in GenBank Accession No. AY251022 and GenBank Accession No. AF079518. SirA was previously identified as a lipoprotein (Heinrichs et al. (1999) J. Bacteriol. 181:1436-1443) and its expression is strictly controlled by the activity of the Fur protein in *S. aureus.* SirB and SirC encode the transmembrane domains of an ABC-transporter. In particular, mutation of *sirA* or *sirB* increases resistance of *S. aureus* to streptonigrin and results in compromised growth in iron-restricted media. Such mutants are also compromised in the ability to recognize and transport the staphylobactin siderophore into the cell. The terms "SirA", "SirB" and "SirC" encompass fragments or portions thereof and biologically active fragments or portions thereof.

The term "SirABC iron-siderophore transport system" refers the SirABC transporter that is comprised of SirA, SirB, SirC, and FhuC polypeptides.

The terms "Sir protein" or "Sir polypeptide" refer to SirA, SirB and/or SirC proteins. The terms "*sir* nucleotide", "*sir* nucleic acid", or "*sir* gene" refer to *sirA*, *sirB* and/or *sirC* nucleic acids.

The term "Sir deficient strain" refers to a bacterial strain that does not express at least one Sir protein. The term "FhuC deficient strain" refers to a bacterial strain that does not express FhuC.

The term "staphylobactin" refers to the iron-siderophore that is transported into cell by the SirABC iron-siderophore transport system.

The term "small molecule" refers to a compound, which has a molecular weight of less than about 5 kD, less than about 2.5 kD, less than about 1.5 kD, or less than about 0.9 kD. Small molecules may be, for example, nucleic acids, peptides, polypeptides, peptide nucleic acids, peptidomimetics, carbohydrates, lipids or other organic (carbon containing) or inorganic molecules. Many pharmaceutical companies have extensive libraries of chemical and/or biological mixtures, often fungal, bacterial, or algal extracts, which can be screened with any of the assays of the invention. The term "small organic molecule" refers to a small molecule that is often identified as being an organic or medicinal compound, and does not include molecules that are exclusively nucleic acids, peptides or polypeptides.

The term "substantially homologous" when used in connection with amino acid sequences, refers to sequences which are substantially identical to or similar in sequence with each other, giving rise to a homology of conformation and thus to retention, to a useful degree, of one or more biological (including immunological) activities. The term is not intended to imply a common evolution of the sequences.

A "subject" refers to a male or female mammal, including humans.

A "vector" is a self-replicating nucleic acid molecule that transfers an inserted nucleic acid molecule into and/or between host cells. The term includes vectors that function primarily for insertion of a nucleic acid molecule into a cell, replication of vectors that function primarily for the replication of nucleic acid, and expression vectors that function for transcription and/or translation of the DNA or RNA. Also included are vectors that provide more than one of the above functions. As used herein, "expression vectors" are defined as polynucleotides which, when introduced into an appropriate host cell, can be transcribed and translated into a polypeptide(s). An "expression system" usually connotes a suitable host cell comprised of an expression vector that can function to yield a desired expression product.

### 3. sirA, sirB, sirC, and fhuC nucleic acids

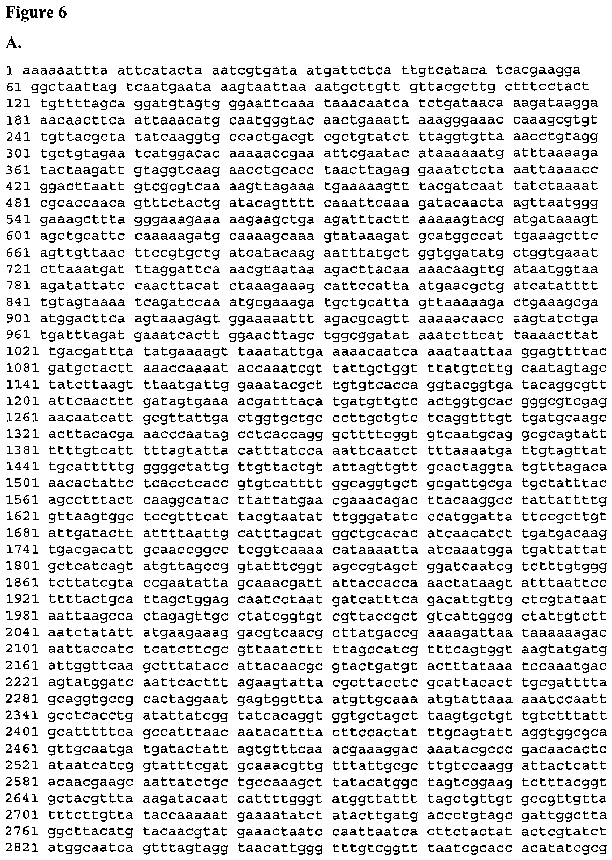

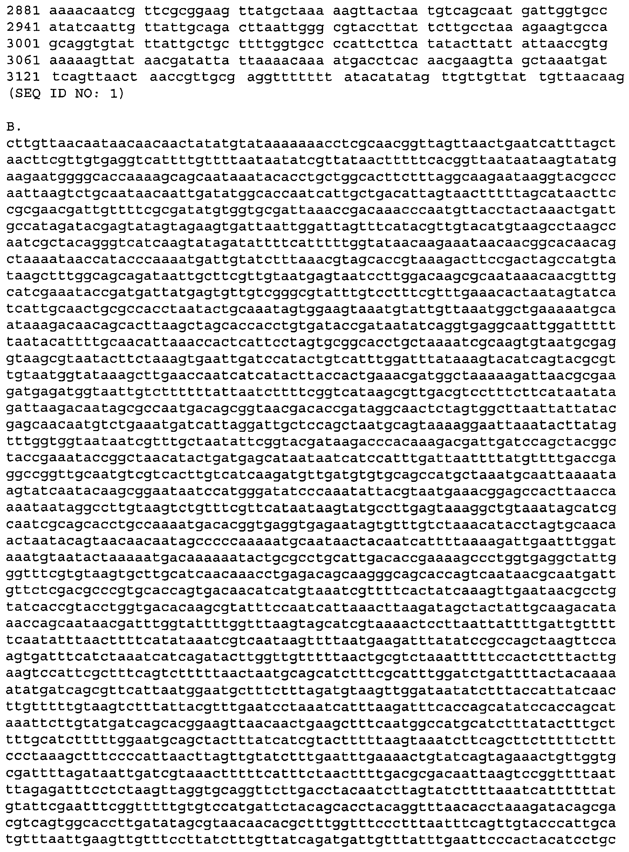

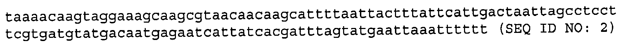

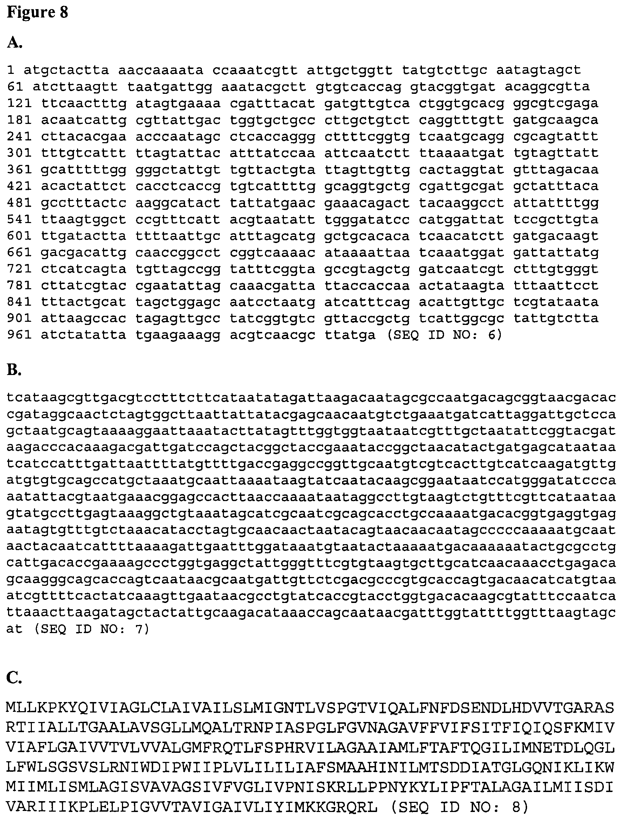

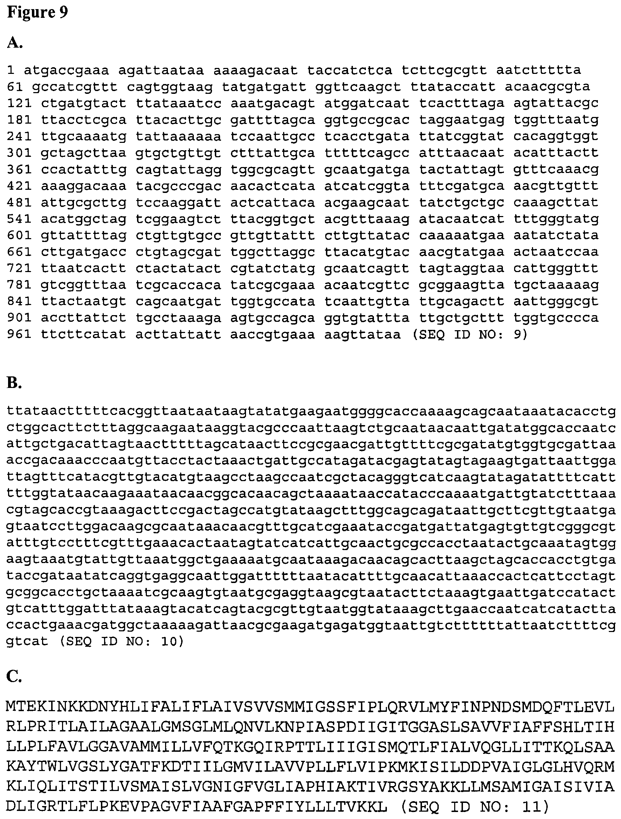

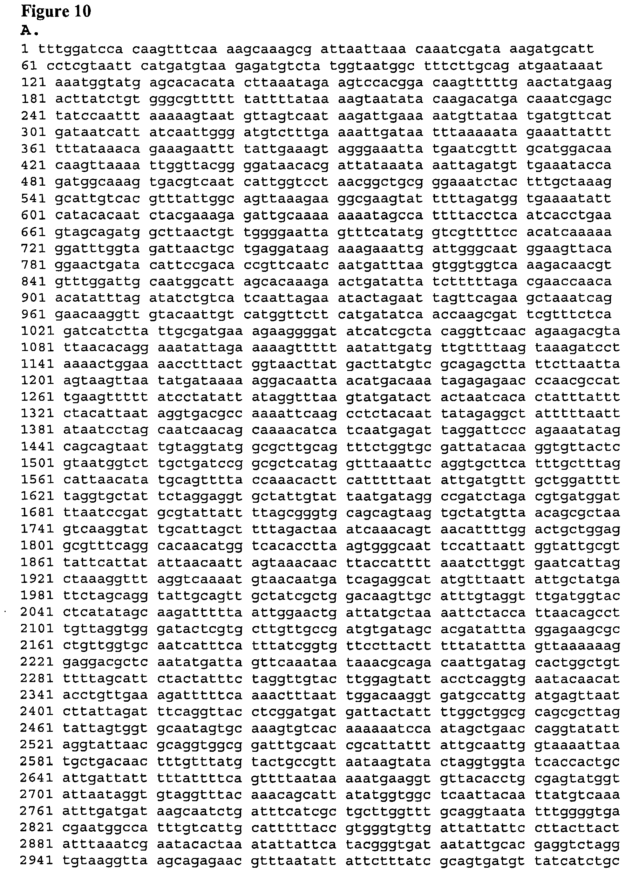

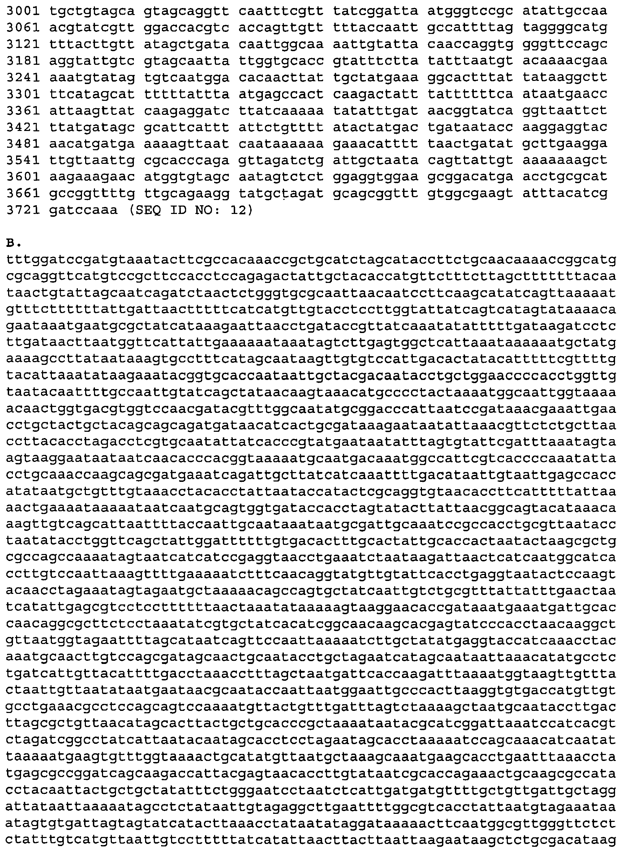

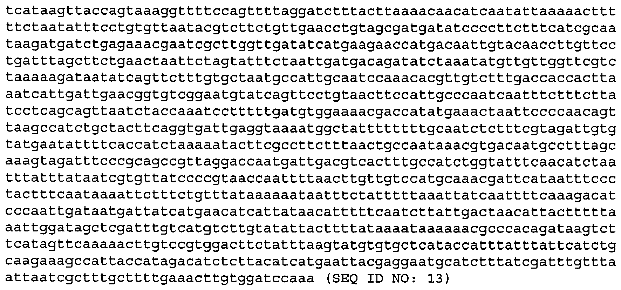

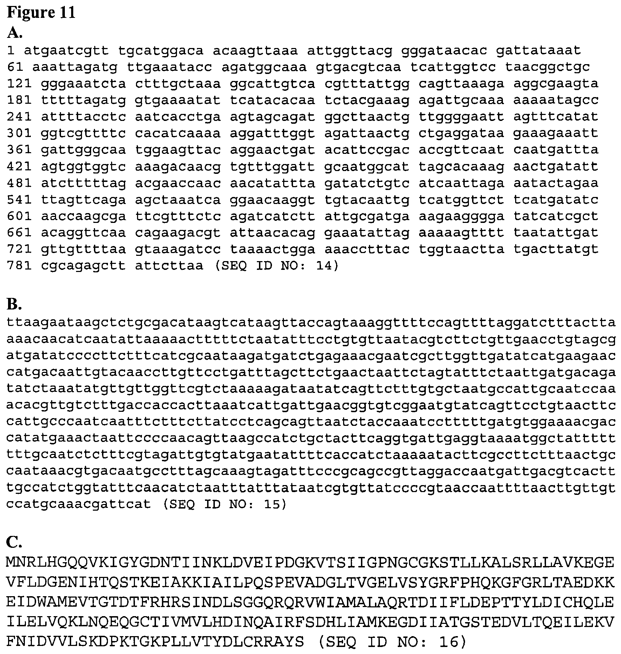

The present disclosure relates to nucleic acid molecules which encode *S. aureus* SirA, SirB, SirC, and FhuC polypeptides, the full complement thereof, or mutants thereof. Figures 6-11 show the nucleic acid sequences that encode SirA, SirB, SirC, and FhuC and the full complement thereof.

Nucleic acids may also comprise, consist of or consist essentially of any of the Sir or FhuC nucleotide sequences or the complement thereof as described herein. Yet other nucleic acids comprise, consist of or consist essentially of a nucleotide sequence that has at least about 70%, 80%, 90%, 95%, 98% or 99% identity or homology with a Sir or FhuC gene or the complement thereof described herein. Substantially homologous sequences may be identified using stringent hybridization conditions.

Isolated nucleic acids which differ from the nucleic acids of the disclosure due to degeneracy in the genetic code are also within the scope of the invention. For example, a number of amino acids are designated by more than one triplet. Codons that specify the same amino acid, or synonyms (for example, CAU and CAC are synonyms for histidine) may result in "silent" mutations which do not affect the amino acid sequence of the protein. However, it is expected that DNA sequence polymorphisms that do lead to changes in the amino acid sequences of the polypeptides of the disclosure will exist. One skilled in the art will appreciate that these variations in one or more nucleotides (from less than 1% up to about 3 or 5% or possibly more of the nucleotides) of the nucleic acids encoding a particular protein of the disclosure may exist among a given species due to natural allelic variation. Any and all such nucleotide variations and resulting amino acid polymorphisms are within the scope of this disclosure.

Nucleic acids encoding proteins which have amino acid sequences evolutionarily related to a polypeptide disclosed herein are provided, wherein "evolutionarily related to", refers to proteins having different amino acid sequences which have arisen naturally (e.g. by allelic variance or by differential splicing), as well as mutational variants of the proteins of the disclosure which are derived, for example, by combinatorial mutagenesis.

Fragments of the polynucleotides of the disclosure encoding a biologically active portion of the subject polypeptides are also provided. As used herein, a fragment of a nucleic acid encoding an active portion of a polypeptide disclosed herein refers to a nucleotide sequence having fewer nucleotides than the nucleotide sequence encoding the full length amino acid sequence of a polypeptide of the disclosure and which encodes a given polypeptide that retains at least a portion of a biological activity of the full-length Sir or FhuC protein as defined herein, or alternatively, which is functional as a modulator of the biological activity of the full-length protein. For example, such fragments include a polypeptide containing a domain of the full-length protein from which the polypeptide is derived that mediates the interaction of the protein with another molecule (e.g., polypeptide, DNA, RNA, etc.).

Nucleic acids provided herein may also contain linker sequences, modified restriction endonuclease sites and other sequences useful for molecular cloning, expression or purification of such recombinant polypeptides.

A nucleic acid encoding a Sir or FhuC polypeptide provided herein may be obtained from mRNA or genomic DNA from any organism in accordance with protocols described herein, as well as those generally known to those skilled in the art. A cDNA encoding a polypeptide of the disclosure, for example, may be obtained by isolating total mRNA from an organism, for example, a bacteria, virus, mammal, etc. Double stranded cDNAs may then be prepared from the total mRNA, and subsequently inserted into a suitable plasmid or bacteriophage vector using any one of a number of known techniques. A gene encoding a polypeptide of the disclosure may also be cloned using established polymerase chain reaction techniques in accordance with the nucleotide sequence information provided by the disclosure. Methods for amplification of a nucleic acid of the disclosure, or a fragment thereof may comprise: (a) providing a pair of single stranded oligonucleotides, each of which is at least eight nucleotides in length, complementary to sequences of a nucleic acid of the disclosure, and wherein the sequences to which the oligonucleotides are complementary are at least ten nucleotides apart; and (b) contacting the oligonucleotides with a sample comprising a nucleic acid comprising the nucleic acid of the disclosure under conditions which permit amplification of the region located between the pair of oligonucleotides, thereby amplifying the nucleic acid.

The present disclosure also features recombinant vectors, which include the isolated *sir* or *fhuC* nucleic acids, and to host cells containing the recombinant vectors, as well as to methods of making such vectors and host cells and for using them for production of *S. aureus* polypeptides by recombinant techniques.

Appropriate vectors may be introduced into host cells using well known techniques such as infection, transduction, transfection, transvection, electroporation and transformation. The vector may be, for example, a phage, plasmid, viral or retroviral vector. Retroviral vectors may be replication competent or replication defective. In the latter case, viral propagation generally will occur only in complementing host cells.

The vector may contain a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells.

Preferred vectors comprise cis-acting control regions to the polynucleotide of interest. Appropriate trans-acting factors may be supplied by the host, supplied by a complementing vector or supplied by the vector itself upon introduction into the host.

The vectors may provide for specific expression, which may be inducible and/or cell type-specific. Particularly preferred among such vectors are those inducible by environmental factors that are easy to manipulate, such as temperature and nutrient additives.

Expression vectors useful in the present invention include chromosomal-, episomal- and virus-derived vectors, *e.g.,* vectors derived from bacterial plasmids, bacteriophage, yeast episomes, yeast chromosomal elements, viruses such as baculoviruses, papova viruses, vaccinia viruses, adenoviruses, fowl pox viruses, pseudorabies viruses and retroviruses, and vectors derived from combinations thereof, such as cosmids and phagemids.

The DNA insert should be operatively linked to an appropriate promoter, such as the phage lambda PL promoter, the *E. coli* lac, trp and tac promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating site at the beginning and a termination codon (UAA, UGA or UAG) appropriately positioned at the end of the polypeptide to be translated.

As indicated, the expression vectors will preferably include at least one selectable marker. Such markers include dihydrofolate reductase or neomycin resistance for eukaryotic cell culture and tetracycline, kanamycin, or ampicillin resistance genes for culturing in *E. coli* and other bacteria. Representative examples of appropriate hosts include, but are not limited to, bacterial cells, such as *E. coli*, *Streptomyces* and *Salmonella typhimurium* cells; fungal cells, such as yeast cells; insect cells such as *Drosophila* S2 and Sf9 cells; animal cells such as CHO, COS and Bowes melanoma cells; and plant cells. Appropriate culture mediums and conditions for the above-described host cells are known in the art.

Among vectors preferred for use in bacteria include pQE70, pQE60 and pQE9, pQE10 available from Qiagen; pBS vectors, Phagescript vectors, Bluescript vectors, pNH8A, pNH16a, pNH18A, pNH46A available from Stratagene; pET series of vectors available from Novagen; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Among known bacterial promoters suitable for use in the present invention include the *E. coli* lacI and lacZ promoters, the T3, T5 and T7 promoters, the gpt promoter, the lambda PR and PL promoters, the trp promoter and the xyI/tet chimeric promoter. Suitable eukaryotic promoters include the CMV immediate early promoter, the HSV thymidine kinase promoter, the early and late SV40 promoters, the promoters of retroviral LTRs, such as those of the Rous sarcoma virus (RSV), and metallothionein promoters, such as the mouse metallothionein-I promoter.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals (for example, Davis, et al., Basic Methods In Molecular Biology (1986)).

Transcription of DNA encoding the polypeptides of the present disclosure by higher eukaryotes may be increased by inserting an enhancer sequence into the vector. Enhancers are cis-acting elements of DNA, usually about from 10 to 300 nucleotides that act to increase transcriptional activity of a promoter in a given host cell-type. Examples of enhancers include the SV40 enhancer, which is located on the late side of the replication origin at nucleotides 100 to 270, the cytomegalovirus early promoter enhancer, the polyoma enhancer on the late side of the replication origin, and adenovirus enhancers.

For secretion of the translated polypeptide into the lumen of the endoplasmic reticulum, into the periplasmic space or into the extracellular environment, appropriate secretion signals may be incorporated into the expressed polypeptide, for example, the amino acid sequence KDEL. The signals may be endogenous to the polypeptide or they may be heterologous signals. Alternatively, as demonstrated in Example 3, *sirA* lacking a signal peptide may be cloned into an *E. coli* expression vector to produce large quantities of soluble SirA.

Coding sequences for a polypeptide of interest may be incorporated as a part of a fusion gene including a nucleotide sequence encoding a different polypeptide. The present disclosure contemplates an isolated nucleic acid comprising a nucleic acid of the disclosure and at least one heterologous sequence encoding a heterologous peptide linked in frame to the nucleotide sequence of the nucleic acid of the disclosure so as to encode a fusion protein comprising the heterologous polypeptide. The heterologous polypeptide may be fused to (a) the C-terminus of the polypeptide encoded by the nucleic acid of the disclosure, (b) the N-terminus of the polypeptide, or (c) the C-terminus and the N-terminus of the polypeptide. In certain instances, the heterologous sequence encodes a polypeptide permitting the detection, isolation, solubilization and/or stabilization of the polypeptide to which it is fused. The heterologous sequence may encode a polypeptide selected from the group consisting of a polyHis tag, myc, HA, GST, protein A, protein G, calmodulin-binding peptide, thioredoxin, maltose-binding protein, poly arginine, poly His-Asp, FLAG, a portion of an immunoglobulin protein, and a transcytosis peptide.

Fusion expression systems can be useful when it is desirable to produce an immunogenic fragment of a polypeptide of the disclosure. For example, the VP6 capsid protein of rotavirus may be used as an immunologic carrier protein for portions of polypeptide, either in the monomeric form or in the form of a viral' particle. The nucleic acid sequences corresponding to the portion of a polypeptide of the disclosure to which antibodies are to be raised may be incorporated into a fusion gene construct which includes coding sequences for a late vaccinia virus structural protein to produce a set of recombinant viruses expressing fusion proteins comprising a portion of the protein as part of the virion. The Hepatitis B surface antigen may also be utilized in this role as well. Similarly, chimeric constructs coding for fusion proteins containing a portion of a polypeptide of the disclosure and the poliovirus capsid protein may be created to enhance immunogenicity (see, for example, EP Publication NO: 0259149; and Evans et al., (1989) Nature 339:385; Huang et al., (1988) J. Virol. 62:3855; and Schlienger et al., (1992) J. Virol. 66:2).

Fusion proteins may facilitate the expression and/or purification of proteins. For example, a polypeptide of the disclosure may be generated as a glutathione-S-transferase (GST) fusion protein. Such GST fusion proteins may be used to simplify purification of a polypeptide of the disclosure, such as through the use of glutathione-derivatized matrices (see, for example, Current Protocols in Molecular Biology, eds. Ausubel et al., (N.Y.: John Wiley & Sons, 1991)). In another embodiment, a fusion gene coding for a purification leader sequence, such as a poly-(His)/enterokinase cleavage site sequence at the N-terminus of the desired portion of the recombinant protein, may allow purification of the expressed fusion protein by affinity chromatography using a Ni²⁺ metal resin. The purification leader sequence may then be subsequently removed by treatment with enterokinase to provide the purified protein (e.g., see Hochuli et al., (1987) J. Chromatography 411: 177; and Janknecht et al., PNAS USA 88:8972).

Techniques for making fusion genes are well known. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene may be synthesized by conventional techniques including automated DNA synthesizers. Alternatively, PCR amplification of gene fragments may be carried out using anchor primers which give rise to complementary overhangs between two consecutive gene fragments which may subsequently be annealed to generate a chimeric gene sequence (see, for example, Current Protocols in Molecular Biology, eds. Ausubel et al., John Wiley & Sons: 1992).

Nucleic acids of the disclosure may be immobilized onto a solid surface, including, plates, microtiter plates, slides, beads, particles, spheres, films, strands, precipitates, gels, sheets, tubing, containers, capillaries, pads, slices, etc. The nucleic acids of the disclosure may be immobilized onto a chip as part of an array. The array may comprise one or more polynucleotides described herein. In one embodiment, the chip comprises one or more polynucleotides of the disclosure as part of an array of polynucleotide sequences.

Also disclosed herein is the use of nucleic acids of the disclosure in "antisense therapy". As used herein, antisense therapy refers to administration or *in situ* generation of oligonucleotide probes or their derivatives which specifically hybridize or otherwise bind under cellular conditions with the cellular mRNA and/or genomic DNA encoding one of the polypeptides of the disclosure so as to inhibit expression of that polypeptide, e.g., by inhibiting transcription and/or translation. The binding may be by conventional base pair complementarity, or, for example, in the case of binding to DNA duplexes, through specific interactions in the major groove of the double helix. In general, antisense therapy refers to the range of techniques generally employed in the art, and includes any therapy which relies on specific binding to oligonucleotide sequences.

The oligonucleotide may be conjugated to another molecule, e.g., a peptide, hybridization triggered cross-linking agent transport agent, hybridization-triggered cleavage agent, etc. An antisense molecule can be a "peptide nucleic acid" (PNA). PNA refers to an antisense molecule or anti-gene agent which comprises an oligonucleotide of at least about 5 nucleotides in length linked to a peptide backbone of amino acid residues ending in lysine. The terminal lysine confers solubility to the composition. PNAs preferentially bind complementary single stranded DNA or RNA and stop transcript elongation, and may be pegylated to extend their lifespan in the cell.

An antisense construct of the present disclosure may be delivered, for example, as an expression plasmid which, when transcribed in the cell, produces RNA which is complementary to at least a unique portion of the mRNA which encodes a polypeptide of the disclosure. Alternatively, the antisense construct may be an oligonucleotide probe which is generated *ex vivo* and which, when introduced into the cell causes inhibition of expression by hybridizing with the mRNA and/or genomic sequences encoding a polypeptide of the disclosure. Such oligonucleotide probes may be modified oligonucleotides which are resistant to endogenous nucleases, e.g., exonucleases and/or endonucleases, and are therefore stable *in vivo.* Exemplary nucleic acid molecules for use as antisense oligonucleotides are phosphoramidate, phosphothioate and methylphosphonate analogs of DNA (see also U.S. Patents 5,176,996; 5,264,564; and 5,256,775). Additionally, general approaches to constructing oligomers useful in antisense therapy have been reviewed, for example, by van der Krol et al., (1988) Biotechniques 6:958-976; and Stein et al., (1988) Cancer Res 48:2659-2668.

Double stranded small interfering RNAs (siRNAs), and methods for administering the same are disclosed. siRNAs decrease or block gene expression. While not wishing to be bound by theory, it is generally thought that siRNAs inhibit gene expression by mediating sequence specific mRNA degradation. RNA interference (RNAi) is the process of sequence-specific, post-transcriptional gene silencing, particularly in animals and plants, initiated by double-stranded RNA (dsRNA) that is homologous in sequence to the silenced gene (Elbashir et al. Nature 2001; 411(6836): 494-8). Accordingly, it is understood that siRNAs and long dsRNAs having substantial sequence identity to all or a portion of a polynucleotide of the present disclosure may be used to inhibit the expression of a nucleic acid of the disclosure.

Alternatively, siRNAs that decrease or block the expression the Sir or FhuC polypeptides described herein may be determined by testing a plurality of siRNA constructs against the target gene. Such siRNAs against a target gene may be chemically synthesized. The nucleotide sequences of the individual RNA strands are selected such that the strand has a region of complementarity to the target gene to be inhibited (i.e., the complementary RNA strand comprises a nucleotide sequence that is complementary to a region of an mRNA transcript that is formed during expression of the target gene, or .its processing products, or a region of a (+) strand virus). The step of synthesizing the RNA strand may involve solid-phase synthesis, wherein individual nucleotides are joined end to end through the formation of internucleotide 3'-5' phosphodiester bonds in consecutive synthesis cycles.

Provided herein are siRNA molecules comprising a nucleotide sequence consisting essentially of a sequence of a Sir or FhuC nucleic acid as described herein. An siRNA molecule may comprise two strands, each strand comprising a nucleotide sequence that is at least essentially complementary to each other, one of which corresponds essentially to a sequence of a target gene. The sequence that corresponds essentially to a sequence of a target gene is referred to as the "sense target sequence" and the sequence that is essentially complementary thereto is referred to as the "antisense target sequence" of the siRNA. The sense and antisense target sequences may be from about 15 to about 30 consecutive nucleotides long; from about 19 to about 25 consecutive nucleotides; from about 19 to 23 consecutive nucleotides or about 19, 20, 21, 22 or 23 nucleotides long. The length of the sense and antisense sequences is determined so that an siRNA having sense and antisense target sequences of that length is capable of inhibiting expression of a target gene, preferably without significantly inducing a host interferon response.

SiRNA target sequences may be predicted using any of the aligorithms provided on the world wide web at the mmcmanus with the extension web.mit.edu/mmcmanus/www/home1.2files/siRNAs.

The sense target sequence may be essentially or substantially identical to the coding or a non-coding portion, or combination thereof, of a target nucleic acid. For example, the sense target sequence may be essentially complementary to the 5' or 3' untranslated region, promoter, intron or exon of a target nucleic acid or complement thereof. It can also be essentially complementary to a region encompassing the border between two such gene regions.

The nucleotide base composition of the sense target sequence can be about 50% adenines (As) and thymidines (Ts) and 50% cytidines (Cs) and guanosines (Gs). Alternatively, the base composition can be at least 50% Cs/Gs, e.g., about 60%, 70% or 80% of Cs/Gs. Accordingly, the choice of sense target sequence may be based on nucleotide base composition. Regarding the accessibility of target nucleic acids by siRNAs, such can be determined, e.g., as described in Lee et al. (2002) Nature Biotech. 19:500. This approach involves the use of oligonucleotides that are complementary to the target nucleic acids as probes to determine substrate accessibility, e.g., in cell extracts. After forming a duplex with the oligonucleotide probe, the substrate becomes susceptible to RNase H. Therefore, the degree of RNase H sensitivity to a given probe as determined, e.g., by PCR, reflects the accessibility of the chosen site, and may be of predictive value for how well a corresponding siRNA would perform in inhibiting transcription from this target gene. One may also use algorithms identifying primers for polymerase chain reaction (PCR) assays or for identifying antisense oligonucleotides for identifying first target sequences.

The sense and antisense target sequences are preferably sufficiently complementary, such that an siRNA comprising both sequences is able to inhibit expression of the target gene, i.e., to mediate RNA interference. For example, the sequences may be sufficiently complementary to permit hybridization under the desired conditions, e.g., in a cell. Accordingly, the sense and antisense target sequences may be at least about 95%, 97%, 98%, 99% or 100% identical and may, e.g., differ in at most 5, 4, 3, 2, 1 or 0 nucleotides.

Sense and antisense target sequences are also preferably sequences that are not likely to significantly interact with sequences other than the target nucleic acid or complement thereof. This can be confirmed by, e.g., comparing the chosen sequence to the other sequences in the genome of the target cell. Sequence comparisons can be performed according to methods known in the art, e.g., using the BLAST algorithm, further described herein. Of course, small scale experiments can also be performed to confirm that a particular first target sequence is capable of specifically inhibiting expression of a target nucleic acid and essentially not that of other genes.

siRNAs may also comprise sequences in addition to the sense and antisense sequences. For example, an siRNA may be an RNA duplex consisting of two strands of RNA, in which at least one strand has a 3' overhang. The other strand can be blunt-ended or have an overhang. In the embodiment in which the RNA molecule is double stranded and both strands comprise an overhang, the length of the overhangs may be the same or different for each strand. In a particular embodiment, an siRNA comprises sense and antisense sequences, each of which are on one RNA strand, consisting of about 19-25 nucleotides which are paired and which have overhangs of from about 1 to about 3, particularly about 2, nucleotides on both 3' ends of the RNA. In order to further enhance the stability of the RNA of the present disclosure, the 3' overhangs can be stabilized against degradation. In one embodiment, the RNA is stabilized by including purine nucleotides, such as adenosine or guanosine nucleotides. Alternatively, substitution of pyrimidine nucleotides by modified analogues, e.g., substitution of uridine 2 nucleotide 3' overhangs by 2'-deoxythymidine is tolerated and does not affect the efficiency of RNAi. The absence of a 2' hydroxyl significantly may also enhance the nuclease resistance of the overhang at least in tissue culture medium. RNA strands of siRNAs may have a 5' phosphate and a 3' hydroxyl group.

In one embodiment, an siRNA molecule comprises two strands of RNA forming a duplex. In another embodiment, an siRNA molecule consists of one RNA strand forming a hairpin loop, wherein the sense and antisense target sequences hybridize and the sequence between the two target sequences is a spacer sequence that essentially forms the loop of the hairpin structure. The spacer sequence may be any combination of nucleotides and any length provided that two complementary oligonucleotides linked by a spacer having this sequence can form a hairpin structure, wherein at least part of the spacer forms the loop at the closed end of the hairpin. For example, the spacer sequence can be from about 3 to about 30 nucleotides; from about 3 to about 20 nucleotides; from about 5 to about 15 nucleotides; from about 5 to about 10 nucleotides; or from about 3 to about 9 nucleotides. The sequence can be any sequence, provided that it does not interfere with the formation of a hairpin structure. In particular, the spacer sequence is preferably not a sequence having any significant homology to the first or the second target sequence, since this might interfere with the formation of a hairpin structure. The spacer sequence is also preferably not similar to other sequences, e.g., genomic sequences of the cell into which the nucleic acid will be introduced, since this may result in undesirable effects in the cell;

A person of skill in the art will understand that when referring to a nucleic acid, e.g., an RNA, the RNA may comprise or consist of naturally occurring nucleotides or of nucleotide derivatives that provide, e.g., more stability to the nucleic acid. Any derivative is permitted provided that the nucleic acid is capable of functioning in the desired fashion. For example, an siRNA may comprise nucleotide derivatives provided that the siRNA is still capable of inhibiting expression of the target gene.

For example, siRNAs may include one or more modified base and/or a backbone modified for stability or for other reasons. For example, the phosphodiester linkages of natural RNA may be modified to include at least one of a nitrogen or sulphur heteroatom. Moreover, siRNA comprising unusual bases, such as inosine, or modified bases, such as tritylated bases, to name just two examples, can be used in the invention. It will be appreciated that a great variety of modifications have been made to RNA that serve many useful purposes known to those of skill in the art. The term siRNA as it is employed herein embraces such chemically, enzymatically or metabolically modified forms of siRNA, provided that it is derived from an endogenous template.

There is no limitation on the manner in which an siRNA may be synthesised. Thus, it may synthesized in vitro or in vivo, using manual and/or automated procedures. In vitro synthesis may be chemical or enzymatic, for example using cloned RNA polymerase (e.g., T3, T7, SP6) for transcription of a DNA (or cDNA) template, or a mixture of both. SiRNAs may also be prepared by synthesizing each of the two strands, e.g., chemically, and hybridizing the two strands to form a duplex. *In vivo*, the siRNA may be synthesized using recombinant techniques well known in the art (see e.g., Sambrook, et al., Molecular Cloning: A Laboratory Manual, Second Edition (1989); DNA Cloning, Volumes I and II (D. N Glover ed. 1985); Oligonucleotide Synthesis (M. J. Gait ed, 1984); Nucleic Acid Hybridisation (B. D. Hames & S. J. Higgins eds. 1984); Transcription and Translation (B. D. Hames & S. J. Higgins eds. 1984); Animal Cell Culture (R. I. Freshney ed. 1986); Immobilised Cells and Enzymes (IRL Press, 1986); B. Perbal, A Practical Guide to Molecular Cloning (1984); the series, Methods in Enzymology (Academic Press, Inc.); Gene Transfer Vectors for Mammalian Cells (J. H. Miller and M. P. Calos eds. 1987, Cold Spring Harbor Laboratory), Methods in Enzymology Vol. 154 and Vol. 155 (Wu and Grossman, and Wu, eds., respectively), Mayer and Walker, eds. (1987), Immunochemical Methods in Cell and Molecular Biology (Academic Press, London), Scopes, (1987), Protein Purification: Principles and Practice, Second Edition (Springer-Verlag, N.Y.),and Handbook of Experimental Immunology, Volumes I-IV (D. M. Weir and C. C. Blackwell eds 1986). For example, bacterial cells can be transformed with an expression vector which comprises the DNA template from which the siRNA is to be derived.

If synthesized outside the cell, the siRNA may be purified prior to introduction into the cell. Purification may be by extraction with a solvent (such as phenol/chloroform) or resin, precipitation (for example in ethanol), electrophoresis, chromatography, or a combination thereof. However, purification may result in loss of siRNA and may therefore be minimal or not carried out at all. The siRNA may be dried for storage or dissolved in an aqueous solution, which may contain buffers or salts to promote annealing, and/or stabilization of the RNA strands.

The double-stranded structure may be formed by a single self-complementary RNA strand or two separate complementary RNA strands.

It is known that mammalian cells can respond to extracellular siRNA and therefore may have a transport mechanism for dsRNA (Asher et al. (1969) Nature 223 715-717). Thus, siRNA may be administered extracellularly into a cavity, interstitial space, into the circulation of a mammal, or introduced orally. Methods for oral introduction include direct mixing of the RNA with food of the mammal, as well as engineered approaches in which a species that is used as food is engineered to express the RNA, then fed to the mammal to be affected. For example, food bacteria, such as *Lactococcus lactis*, may be transformed to produce the dsRNA (see WO93/17117, WO97/14806). Vascular or extravascular circulation, the blood or lymph systems and the cerebrospinal fluid are sites where the RNA may be injected.

RNA may be introduced into the cell intracellularly. Physical methods of introducing nucleic acids may also be used in this respect. siRNA may be administered using the microinjection techniques described in Zernicka-Goetz et al. (1997) Development 124, 1133-1137 and Wianny et al. (1998) Chromosoma 107,430-439.

Other physical methods of introducing nucleic acids intracellularly include bombardment by particles covered by the siRNA, for example gene gun technology in which the siRNA is immobilized on gold particles and fired directly at the site of wounding. Thus, the disclosure provides the use of an siRNAin a gene gun for inhibiting the expression of a target gene. Further, there is provided a composition suitable for gene gun therapy comprising an siRNA and gold particles. An alternative physical method includes electroporation of cell membranes in the presence of the siRNA. This method permits RNAi on a large scale. Other methods known in the art for introducing nucleic acids to cells may be used, such as lipid-mediated carrier transport, chemical-mediated transport, such as calcium phosphate, and the like. siRNA may be introduced along with components that perform one or more of the following activities: enhance RNA uptake by the cell, promote annealing of the duplex strands, stabilize the annealed strands, or otherwise increase inhibition of the target gene.

Any known gene therapy technique can be used to administer the RNA. A viral construct packaged into a viral particle would accomplish both efficient introduction of an expression construct into the cell and transcription of siRNA encoded by the expression construct. Thus, siRNA can also be produced inside a cell. Vectors, e.g., expression vectors that comprise a nucleic acid encoding one or the two strands of an siRNA molecule may be used for that purpose. The nucleic acid may further comprise an antisense sequence that is essentially complementary to the sense target sequence. The nucleic acid may further comprise a spacer sequence between the sense and the antisense target sequence. The nucleic acid may further comprise a promoter for directing expression of the sense and antisense sequences in a cell, e.g., an RNA Polymerase II or III promoter and a transcriptional termination signal. The sequences may be operably linked.

In one embodiment a nucleic acid comprises an RNA coding region (e.g., sense or antisense target sequence) operably linked to an RNA polymerase III promoter. The RNA coding region can be immediately followed by a pol III terminator sequence, which directs termination of RNA synthesis by pol III. The pol III terminator sequences generally have 4 or more consecutive thymidine ("T") residues. In a preferred embodiment, a cluster of 5 consecutive T residues is used as the terminator by which pol III transcription is stopped at the second or third T of the DNA template, and thus only 2 to 3 uridine ("U") residues are added to the 3' end of the coding sequence. A variety of pol III promoters can be used with the invention, including for example, the promoter fragments derived from H1 RNA genes or U6 snRNA genes of human or mouse origin or from any other species. In addition, pol III promoters can be modified/engineered to incorporate other desirable properties such as the ability to be induced by small chemical molecules, either ubiquitously or in a tissue-specific manner. For example, in one embodiment the promoter may be activated by tetracycline. In another embodiment the promoter may be activated by IPTG (lacI system).

siRNAs can be produced in cells by transforming cells with two nucleic acids, e.g., vectors, each nucleic acid comprising an expressing cassette, each expression cassette comprising a promoter, an RNA coding sequence (one being a sense target sequence and the other being an antisense target sequence) and a termination signal. Alternatively, a single nucleic acid may comprise these two expression cassettes. In yet another embodiment, a nucleic acid encodes a single stranded RNA comprising a sense target sequence linked to a spacer linked to an antisense target sequence. The nucleic acids may be present in a vector, such as an expression vector, e.g., a eukaryotic expression vector that allows expression of the sense and antisense target sequences in cells into which it is introduced.

Vectors for producing siRNAs are described, e.g., in Paul et al. (2002) Nature Biotechnology 29:505; Xia et al. (2002) Nature Biotechnology 20:1006; Zeng et al. (2002) Mol. Cell 9:1327; Thijn et al. (2002) Science 296:550; BMC Biotechnol. 2002 Aug 28;2(1):15; Lee et al. (2002) Nature Biotechnology 19: 500; McManus et al. (2002) RNA 8:842; Miyagishi et al. (2002) Nature Biotechnology 19:497; Sui et al. (2002) PNAS 99:5515; Yu et al. (2002) PNAS 99:6047; Shi et al. (2003) Trends Genet. 19(1):9; Gaudilliere et al. (2002) J. Biol. Chem. 277(48):46442; US2002/0182223; US 2003/0027783; WO 01/36646 and WO 03/006477. Vectors are also available commercially. For example, the pSilencer is available from Gene Therapy Systems, Inc. and pSUPER RNAi system is available from Oligoengine.

Also provided herein are compositions comprising one or more siRNA or nucleic acid encoding an RNA coding region of an siRNA. Compositions may be pharmaceutical compositions and comprise a pharmaceutically acceptable carrier. Compositions may also be provided in a device for administering the composition in a cell or in a subject. For example a composition may be present in a syringe or on a stent. A composition may also comprise agents facilitating the entry of the siRNA or nucleic acid into a cell.

In general, the oligonucleotides may be synthesized using protocols known in the art, for example, as described in Caruthers et al., Methods in Enzymology (1992) 211:3-19; Thompson et al., International PCT Publication No. WO 99/54459; Wincott et al., Nucl. Acids Res. (1995) 23:2677-2684; Wincott et al., Methods Mol. Bio., (1997) 74:59; Brennan et al., Biotechnol. Bioeng. (1998) 61:33-45; and Brennan, U.S. Pat. No. 6,001,311.

In general, the synthesis of oligonucleotides involves conventional nucleic acid protecting and coupling groups, such as dimethoxytrityl at the 5'-end, and phosphoramidites at the 3'-end. In a non-limiting example, small scale syntheses are conducted on a Expedite 8909 RNA synthesizer sold by Applied Biosystems, Inc. (Weiterstadt, Germany), using ribonucleoside phosphoramidites sold by ChemGenes Corporation (Ashland Technology Center, 200 Homer Avenue, Ashland, MA 01721, USA). Alternatively, syntheses can be performed on a 96-well plate synthesizer, such as the instrument produced by Protogene (Palo Alto, Calif., USA), or by methods such as those described in Usman et al., J. Am. Chem. Soc. (1987) 109:7845; Scaringe et al., Nucl. Acids Res. (1990) 18:5433; Wincott et al., Nucl. Acids Res. (1990) 23:2677-2684; and Wincott et al., Methods Mol. Bio. (1997) 74:59.

The nucleic acid molecules of the present disclosure may be synthesized separately and dsRNAs may be formed post-synthetically, for example, by ligation (Moore et al., Science (1992) 256:9923; Draper et al., International PCT publication No. WO 93/23569; Shabarova et al., Nucl. Acids Res. (1991) 19:4247; Bellon et al., Nucleosides & Nucleotides (1997) 16:951; and Bellon et al., Bioconjugate Chem. (1997) 8:204; or by hybridization following synthesis and/or deprotection. The nucleic acid molecules can be purified by gel electrophoresis using conventional methods or can be purified by high pressure liquid chromatography (HPLC; see Wincott *et al.,* supra, the totality of which is hereby incorporated herein by reference) and re-suspended in water.

In another embodiment, the level of a particular mRNA or polypeptide in a cell is reduced by introduction of a ribozyme into the cell or nucleic acid encoding such. Ribozyme molecules designed to catalytically cleave mRNA transcripts can also be introduced into, or expressed, in cells to inhibit expression of gene Y (*see, e.g.*, Sarver et al., 1990, Science 247:1222-1225 and U.S. Patent No. 5,093,246). One commonly used ribozyme motif is the hammerhead, for which the substrate sequence requirements are minimal. Design of the hammerhead ribozyme is disclosed in Usman et al., Current Opin. Struct. Biol. (1996) 6:527-533. Usman also discusses the therapeutic uses of ribozymes. Ribozymes can also be prepared and used as described in Long et al., FASEB J. (1993) 7:25; Symons, Ann. Rev. Biochem. (1992) 61:641; Perrotta et al., Biochem. (1992) 31:16-17; Ojwang et al., Proc. Natl. Acad. Sci. (USA) (1992) 89:10802-10806; and U.S. Patent No. 5,254,678. Ribozyme cleavage of HIV-I RNA is described in U.S. Patent No. 5,144,019; methods of cleaving RNA using ribozymes is described in U.S. Patent No. 5,116,742; and methods for increasing the specificity of ribozymes are described in U.S. Patent No. 5,225,337 and Koizumi et al., Nucleic Acid Res. (1989) 17:7059-7071. Preparation and use of ribozyme fragments in a hammerhead structure are also described by Koizumi et al., Nucleic Acids Res. (1989) 17:7059-7071. Preparation and use of ribozyme fragments in a hairpin structure are described by Chowrira and Burke, Nucleic Acids Res. (1992) 20:2835. Ribozymes can also be made by rolling transcription as described in Daubendiek and Kool, Nat. Biotechnol. (1997) 15(3):273 -277.

Gene expression can be reduced by targeting deoxyribonucleotide sequences complementary to the regulatory region of the target gene (i.e., the gene promoter and/or enhancers) to form triple helical structures that prevent transcription of the gene in target cells in the body. (See generally, Helene (1991) Anticancer Drug Des., 6(6):569-84; Helene et al. (1992) Ann. N.Y. Acad. Sci., 660:27-36; and Maher (1992) Bioassays 14(12):807-15).

In a further embodiment, RNA aptamers can be introduced into or expressed in a cell. RNA aptamers are specific RNA ligands for proteins, such as for Tat and Rev RNA (Good et al. (1997) Gene Therapy 4: 45-54) that can specifically inhibit their translation.

### 4. SirA, SirB, SirC, and FhuC polypeptides

SirA, SirB, SirC, and FhuC polypeptides described herein include naturally purified products, products of chemical synthetic procedures, and products produced by recombinant techniques from a prokaryotic or eukaryotic host cell, including, for example, bacterial, yeast, higher plant, insect and mammalian cells. In certain embodiments, the polypeptides disclosed herein inhibit the function of Sir polypeptides and FhuC.

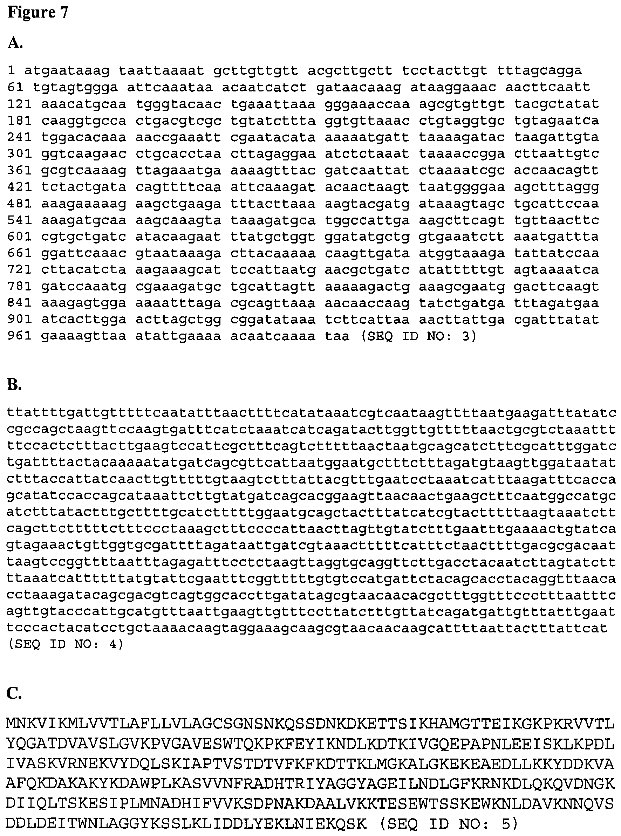

Polypeptides may also comprise, consist of or consist essentially of an amino acid sequence encoded by a nucleotide sequence as shown in Figures 6-11. Exemplary polypeptide sequences for SirA, SirB, SirC, and FhuC are shown in Figures 7-9 and 11, respectively. Yet other polypeptides comprise, consist of or consist essentially of an amino acid sequence that has at least about 70%, 80%, 90%, 95%, 98% or 99% identity or homology with the Sir or FhuC polypeptides described herein. For example, polypeptides that differ from a sequence in a naturally occurring protein in about 1, 2, 3, 4, 5 or more amino acids are also contemplated. The differences may be substitutions, *e.g.,* conservative substitutions, deletions or additions. The differences are preferably in regions that are not significantly conserved among different species. Such regions can be identified by aligning the amino acid sequences from various species. These amino acids can be substituted, *e.g.,* with those found in another species. Other amino acids that may be substituted, inserted or deleted at these or other locations can be identified by mutagenesis studies coupled with biological assays.

Other proteins that are encompassed herein are those that comprise modified amino acids. Exemplary proteins are derivative proteins that may be one modified by glycosylation, pegylation, phosphorylation or any similar process that retains at least one biological function of the protein from which it was derived.

Proteins may also comprise one or more non-naturally occurring amino acids. For example, nonclassical amino acids or chemical amino acid analogs can be introduced as a substitution or addition into proteins. Non-classical amino acids include, but are not limited to, the D-isomers of the common amino acids, 2,4-diaminobutyric acid, alpha-amino isobutyric acid, 4-aminobutyric acid, Abu, 2-amino butyric acid, gamma-Abu, epsilon-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, beta-alanine, fluoro-amino acids, designer amino acids such as beta-methyl amino acids, Calpha-methyl amino acids, Nalpha-methyl amino acids, and amino acid analogs in general. Furthermore, the amino acid can be D (dextrorotary) or L (levorotary).

In certain embodiments, a Sir or FhuC polypeptide described herein may be a fusion protein containing a domain which increases its solubility and/or facilitates its purification, identification, detection, and/or structural characterization. Exemplary domains, include, for example, glutathione S-transferase (GST), protein A, protein G, calmodulin-binding peptide, thioredoxin, maltose binding protein, HA, myc, poly arginine, poly His, poly His-Asp or FLAG fusion proteins and tags. Additional exemplary domains include domains that alter protein localization *in vivo*, such as signal peptides, type III secretion system-targeting peptides, transcytosis domains, nuclear localization signals, etc. In various embodiments, a polypeptide of the disclosure, may comprise one or more heterologous fusions. Polypeptides may contain multiple copies of the same fusion domain or may contain fusions to two or more different domains. The fusions may occur at the N-terminus of the polypeptide, at the C-terminus of the polypeptide, or at both the N- and C-terminus of the polypeptide. It is also within the scope of the invention to include linker sequences between a polypeptide of the disclosure and the fusion domain in order to facilitate construction of the fusion protein or to optimize protein expression or structural constraints of the fusion protein. In another embodiment, the polypeptide may be constructed so as to contain protease cleavage sites between the fusion polypeptide and polypeptide of the disclosure in order to remove the tag after protein expression or thereafter. Examples of suitable endoproteases, include, for example, Factor Xa and TEV proteases.

The *S. aureus* polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, lectin chromatography and high performance liquid chromatography ("HPLC") is employed for purification. Proteins may be used as a substantially pure preparation, e.g., wherein at least about 90% of the protein in the preparation are the desired protein. Compositions comprising at least about 50%, 60%, 70%, or 80% of the desired protein may also be used.

Polypeptides of the disclosure may be synthesized chemically, ribosomally in a cell free system, or ribosomally within a cell. Chemical synthesis of polypeptides of the disclosure may be carried out using a variety of art recognized methods, including stepwise solid phase synthesis, semi-synthesis through the conformationally-assisted re-ligation of peptide fragments, enzymatic ligation of cloned or synthetic peptide segments, and chemical ligation. Native chemical ligation employs a chemoselective reaction of two unprotected peptide segments to produce a transient thioester-linked intermediate. The transient thioester-linked intermediate then spontaneously undergoes a rearrangement to provide the full length ligation product having a native peptide bond at the ligation site. Full length ligation products are chemically identical to proteins produced by cell free synthesis. Full length ligation products may be refolded and/or oxidized, as allowed, to form native disulfide-containing protein molecules. (see e.g., U.S. Patent Nos. 6,184,344 and 6,174,530; and Muir et al., Curr. Opin. Biotech. (1993): vol. 4, p 420; Miller et al., Science (1989): vol. 246, p 1149; Wlodawer et al., Science (1989): vol. 245, p 616; Huang et al., Biochemistry (1991): vol. 30, p 7402; Schnolzer, et al., Int. J. Pept. Prot. Res. (1992): vol. 40, p 180-193; Rajarathnam et al., Science (1994): vol. 264, p 90; R. E. Offord, "Chemical Approaches to Protein Engineering", in Protein Design and the Development of New therapeutics and Vaccines, J. B. Hook, G. Poste, Eds., (Plenum Press, New York; 1990) pp. 253-282; Wallace et al., J. Biol. Chem. (1992): vol. 267, p 3852; Abrahmsen et al., Biochemistry (1991): vol. 30, p 4151; Chang, et al., Proc. Natl. Acad. Sci. USA (1994) 91: 12544-12548; Schnlzer et al., Science (1992): vol., 3256, p 221; and Akaji et al., Chem, Pharm. Bull. (Tokyo) (1985) 33: 184).

It may be advantageous to provide naturally-occurring or experimentally-derived homologs of a polypeptide of the disclosure. Such homologs may function in a limited capacity as a modulator to promote or inhibit a subset of the biological activities of the naturally-occurring form of the polypeptide. Thus, specific biological effects may be elicited by treatment with a homolog of limited function, and with fewer side effects relative to treatment with agonists or antagonists which are directed to all of the biological activities of a polypeptide of the disclosure. For instance, antagonistic homologs may be generated which interfere with the ability of the wild-type polypeptide of the disclosure to associate with certain proteins, but which do not substantially interfere with the formation of complexes between the native polypeptide and other cellular proteins.

Polypeptides may be derived from the full-length polypeptides of the disclosure, Isolated peptidyl portions of those polypeptides may be obtained by screening polypeptides recombinantly produced from the corresponding fragment of the nucleic acid encoding such polypeptides. In addition, fragments may be chemically synthesized using techniques known in the art such as conventional Merrifield solid phase f-Moc or t-Boc chemistry. For example, proteins may be arbitrarily divided into fragments of desired length with no overlap of the fragments, or may be divided into overlapping fragments of a desired length. The fragments may be produced (recombinantly or by chemical synthesis) and tested to identify those peptidyl fragments having a desired property, for example, the capability of functioning as a modulator of the polypeptides of the disclosure, In an illustrative embodiment, peptidyl portions of a protein of the disclosure may be tested for binding activity, as well as inhibitory ability, by expression as, for example, thioredoxin fusion proteins, each of which contains a discrete fragment of a protein of the disclosure (see, for example, U.S. Patents 5,270,181 and 5,292,646; and PCT publication WO94/02502).

Truncated polypeptides may be prepared. Truncated polypeptides have from 1 to 20 or more amino acid residues removed from either or both the N- and C-termini. Such truncated polypeptides may prove more amenable to expression, purification or characterization than the full-length polypeptide. For example, truncated polypeptides may prove more amenable than the full-length polypeptide to crystallization, to yielding high quality diffracting crystals or to yielding an HSQC spectrum with high intensity peaks and minimally overlapping peaks. In addition, the use of truncated polypeptides may also identify stable and active domains of the full-length polypeptide that may be more amenable to characterization.

It is also possible to modify the structure of the polypeptides of the disclosure for such purposes as enhancing therapeutic or prophylactic efficacy, or stability (e.g., *ex vivo* shelf life, resistance to proteolytic degradation *in vivo*, etc.). Such modified polypeptides, when designed to retain at least one activity of the naturally-occurring form of the protein, are considered "functional equivalents" of the polypeptides described in more detail herein. Such modified polypeptides may be produced, for instance, by amino acid substitution, deletion, or addition, which substitutions may consist in whole or part by conservative amino acid substitutions.

For instance, it is reasonable to expect that an isolated conservative amino acid substitution, such as replacement of a leucine with an isoleucine or valine, an aspartate with a glutamate, a threonine with a serine, will not have a major affect on the biological activity of the resulting molecule. Whether a change in the amino acid sequence of a polypeptide results in a functional homolog may be readily determined by assessing the ability of the variant polypeptide to produce a response similar to that of the wild-type protein. Polypeptides in which more than one replacement has taken place may readily be tested in the same manner.

Methods of generating sets of combinatorial mutants of polypeptides of the disclosure are provided, as well as truncation mutants, and is especially useful for identifying potential variant sequences (e.g., homologs). The purpose of screening such combinatorial libraries is to generate, for example, homologs which may modulate the activity of a polypeptide of the disclosure, or alternatively, which possess novel activities altogether. Combinatorially-derived homologs may be generated which have a selective potency relative to a naturally-occurring protein. Such homologs may be used in the development of therapeutics.

Likewise, mutagenesis may give rise to homologs which have intracellular half-lives dramatically different than the corresponding wild-type protein. For example, the altered protein may be rendered either more stable or less stable to proteolytic degradation or other cellular process which result in destruction of, or otherwise inactivation of the protein. Such homologs, and the genes which encode them, may be utilized to alter protein expression by modulating the half-life of the protein. As above, such proteins may be used for the development of therapeutics or treatment.

In similar fashion, protein homologs may be generated by the present combinatorial approach to act as antagonists, in that they are able to interfere with the activity of the corresponding wild-type protein.

In a representative example of this method, the amino acid sequences for a population of protein homologs are aligned, preferably to promote the highest homology possible. Such a population of variants may include, for example, homologs from one or more species, or homologs from the same species but which differ due to mutation. Amino acids which appear at each position of the aligned sequences are selected to create a degenerate set of combinatorial sequences. The combinatorial library may be produced by way of a degenerate library of genes encoding a library of polypeptides which each include at least a portion of potential protein sequences. For instance, a mixture of synthetic oligonucleotides may be enzymatically ligated into gene sequences such that the degenerate set of potential nucleotide sequences are expressible as individual polypeptides, or alternatively, as a set of larger fusion proteins (e.g. for phage display).

There are many ways by which the library of potential homologs may be generated from a degenerate oligonucleotide sequence. Chemical synthesis of a degenerate gene sequence may be carried out in an automatic DNA synthesizer, and the synthetic genes may then be ligated into an appropriate vector for expression. One purpose of a degenerate set of genes is to provide, in one mixture, all of the sequences encoding the desired set of potential protein sequences. The synthesis of degenerate oligonucleotides is well known in the art (see for example, Narang (1983) Tetrahedron 39:3; Itakura et al. (1981) Recombinant DNA, Proc. 3rd Cleveland Sympos. Macromolecules, ed. AG Walton, Amsterdam: Elsevier pp. 273-289; Itakura et al. (1984) Annu. Rev. Biochem. 53:323; Itakura et al. (1984) Science 198:1056; Ike et al., (1983) Nucleic Acid Res. 11:477). Such techniques have been employed in the directed evolution of other proteins (see, for example, Scott et al. (1990) Science 249:386-390; Roberts et al. (1992) PNAS USA 89:2429-2433; Devlin et al. (1990) Science 249: 404-406; Cwirla et al. (1990) PNAS USA 87: 6378-6382; as well as U.S. Patent Nos: 5,223,409, 5,198,346, and 5,096,815).

Alternatively, other forms of mutagenesis may be utilized to generate a combinatorial library. For example, protein homologs (both agonist and antagonist forms) may be generated and isolated from a library by screening using, for example, alanine scanning mutagenesis and the like (Ruf et al. (1994) Biochemistry 33:1565-1572; Wang et al. (1994) J. Biol. Chem. 269:3095-3099; Balint et al. (1993) Gene 137:109-118; Grodberg et al. (1993) Eur. J. Biochem. 218:597-601; Nagashima et al. (1993) J. Biol. Chem. 268:2888-2892; Lowman et al. (1991) Biochemistry 30:10832-10838; and Cunningham et al., (1989) Science 244:1081-1085), by linker scanning mutagenesis (Gustin et al. (1993) Virology 193:653-660; Brown et al. (1992) Mol. Cell Biol. 12:2644-2652; McKnight et al. (1982) Science 232:316); by saturation mutagenesis (Meyers et al. (1986) Science 232:613); by PCR mutagenesis (Leung et al. (1989) Method Cell Mol Biol 1:11-19); or by random mutagenesis (Miller et al. (1992) A Short Course in Bacterial Genetics, CSHL Press, Cold Spring Harbor, NY; and Greener et al. (1994) Strategies in Mol Biol 7:32-34). Linker scanning mutagenesis, particularly in a combinatorial setting, is an attractive method for identifying truncated forms of proteins that are bioactive.

A wide range of techniques are known in the art for screening gene products of combinatorial libraries made by point mutations and truncations, and for screening cDNA libraries for gene products having a certain property. Such techniques will be generally adaptable for rapid screening of the gene libraries generated by the combinatorial mutagenesis of protein homologs. The most widely used techniques for screening large gene libraries typically comprises cloning the gene library into replicable expression vectors, transforming appropriate cells with the resulting library of vectors, and expressing the combinatorial genes under conditions in which detection of a desired activity facilitates relatively easy isolation of the vector encoding the gene whose product was detected.

In an illustrative embodiment of a screening assay, candidate combinatorial gene products are displayed on the surface of a cell and the ability of particular cells or viral particles to bind to the combinatorial gene product is detected in a "panning assay". For instance, the gene library may be cloned into the gene for a surface membrane protein of a bacterial cell (Ladner et al., WO 88/06630; Fuchs et al., (1991) Bio/Technology 9:1370-1371; and Goward et al., (1992) TIBS 18:136-140), and the resulting fusion protein detected by panning, e.g. using a fluorescently labeled molecule which binds the cell surface protein, e.g. FITC-substrate, to score for potentially functional homologs. Cells may be visually inspected and separated under a fluorescence microscope, or, when the morphology of the cell permits, separated by a fluorescence-activated cell sorter. This method may be used to identify substrates or other polypeptides that can interact with a polypeptide of the disclosure.

In similar fashion, the gene library may be expressed as a fusion protein on the surface of a viral particle. For instance, in the filamentous phage system, foreign peptide sequences may be expressed on the surface of infectious phage, thereby conferring two benefits. First, because these phage may be applied to affinity matrices at very high concentrations, a large number of phage may be screened at one time. Second, because each infectious phage displays the combinatorial gene product on its surface, if a particular phage is recovered from an affinity matrix in low yield, the phage may be amplified by another round of infection. The group of almost identical *E. coli* filamentous phages M13, fd, and f1 are most often used in phage display libraries, as either of the phage gIII or gVIII coat proteins may be used to generate fusion proteins without disrupting the ultimate packaging of the viral particle (Ladner et al., PCT publication WO 90/02909; Garrard et al., PCT publication WO 92/09690; Marks et al., (1992) J. Biol. Chem. 267:16007-16010; Griffiths et al., (1993) EMBO J. 12:725-734; Clackson et al., (1991) Nature 352:624-628; and Barbas et al., (1992) PNAS USA 89:4457-4461). Other phage coat proteins may be used as appropriate.

The polypeptides disclosed herein may be reduced to generate mimetics, e.g. peptide or non-peptide agents, which are able to mimic binding of the authentic protein to another cellular partner. Such mutagenic techniques as described above, as well as the thioredoxin system, are also particularly useful for mapping the determinants of a protein which participates in a protein-protein interaction with another protein. To illustrate, the critical residues of a protein which are involved in molecular recognition of a substrate protein may be determined and used to generate peptidomimetics that may bind to the substrate protein. The peptidomimetic may then be used as an inhibitor of the wild-type protein by binding to the substrate and covering up the critical residues needed for interaction with the wild-type protein, thereby preventing interaction of the protein and the substrate. By employing, for example, scanning mutagenesis to map the amino acid residues of a protein which are involved in binding a substrate polypeptide, peptidomimetic compounds may be generated which mimic those residues in binding to the substrate.

For instance, derivatives of the Sir proteins and FhuC protein described herein may be chemically modified peptides and peptidomimetics. Peptidomimetics are compounds based on, or derived from, peptides and proteins. Peptidomimetics can be obtained by structural modification of known peptide sequences using unnatural amino acids, conformational restraints, isosteric replacement, and the like. The subject peptidomimetics constitute the continum of structural space between peptides and non-peptide synthetic structures; peptidomimetics may be useful, therefore, in delineating pharmacophores and in helping to translate peptides into nonpeptide compounds with the activity of the parent peptides.

Moreover, mimetopes of the subject peptides can be provided. Such peptidomimetics can have such attributes as being non-hydrolyzable (e.g., increased stability against proteases or other physiological conditions which degrade the corresponding peptide), increased specificity and/or potency for stimulating cell differentiation. For illustrative purposes, non-hydrolyzable peptide analogs of such residues may be generated using benzodiazepine (e.g., see Freidinger et al., in Peptides: Chemistry and Biology, G.R. Marshall ed., ESCOM Publisher: Leiden, Netherlands, 1988), azepine (e.g., see Huffman et al., in Peptides: Chemistry and Biology, G.R. Marshall ed., ESCOM Publisher: Leiden, Netherlands, 1988), substituted gamma lactam rings (Garvey et al., in Peptides: Chemistry and Biology, G.R. Marshall ed., ESCOM Publisher: Leiden, Netherlands, 1988), keto-methylene pseudopeptides (Ewenson et al., (1986) J. Med. Chem. 29:295; and Ewenson et al., in Peptides: Structure and Function (Proceedings of the 9th American Peptide Symposium) Pierce Chemical Co. Rockland, IL, 1985), β-turn dipeptide cores (Nagai et al., (1985) Tetrahedron Lett 26:647; and Sato et al., (1986) J Chem Soc Perkin Trans 1:1231), and β-aminoalcohols (Gordon et al., (1985) Biochem Biophys Res Commun 126:419; and Dann et al., (1986) Biochem Biophys Res Commun 134:71).

In addition to a variety of sidechain replacements which can be carried out to generate peptidomimetics, the description specifically contemplates the use of conformationally restrained mimics of peptide secondary structure. Numerous surrogates have been developed for the amide bond of peptides. Frequently exploited surrogates for the amide bond include the following groups (i) trans-olefins, (ii) fluoroalkene, (iii) methyleneamino, (iv) phosphonamides, and (v) sulfonamides.

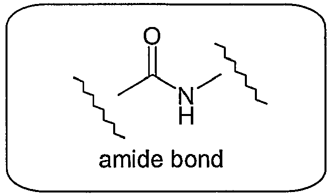

### Examples of Surrogates:

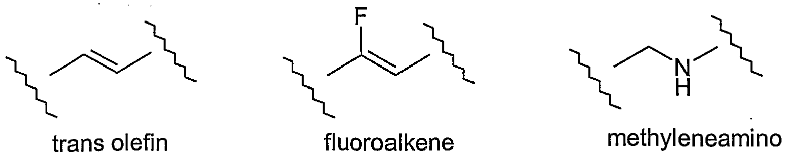

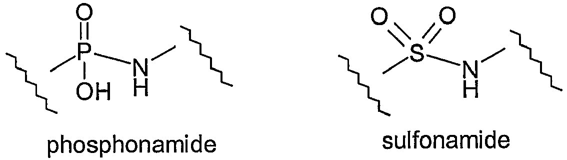

Additionally, peptidomimietics based on more substantial modifications of the backbone of a peptide can be used. Peptidomimetics which fall in this category include (i) retro-inverso analogs, and (ii) N-alkyl glycine analogs (so-called peptoids).

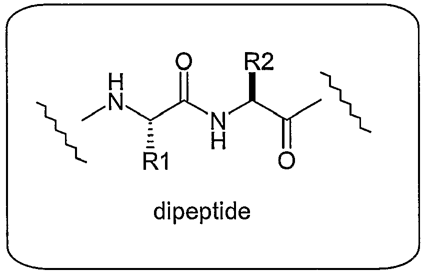

### Examples of analogs:

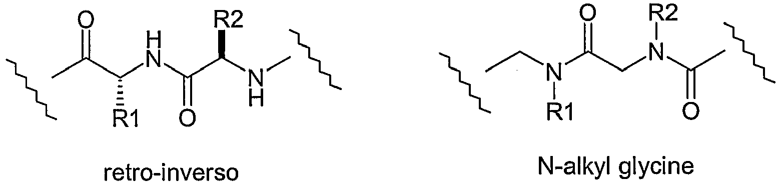

Furthermore, the methods of combinatorial chemistry are being brought to bear, on the development of new peptidomimetics. For example, one embodiment of a so-called "peptide morphing" strategy focuses on the random generation of a library of peptide analogs that comprise a wide range of peptide bond substitutes.

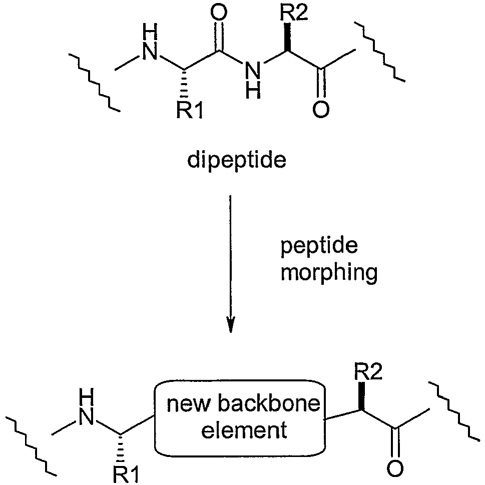

In an exemplary embodiment, the peptidomimetic can be derived as a retro-inverso analog of the peptide. Such retro-inverso analogs can be made according to the methods known in the art, such as that described by the Sisto et al. U.S. Patent 4,522,752. A retro-inverso analog can be generated as described, e.g., in WO 00/01720. It will be understood that a mixed peptide, e.g. including some normal peptide linkages, may be generated. As a general guide, sites which are most susceptible to proteolysis are typically altered, with less susceptible amide linkages being optional for mimetic switching. The final product, or intermediates thereof, can be purified by HPLC.

Peptides may comprise at least one amino acid or every amino acid that is a D stereoisomer. Other peptides may comprise at least one amino acid that is reversed. The amino acid that is reversed may be a D stereoisomer. Every amino acid of a peptide may be reversed and/or every amino acid may be a D stereoisomer.

In another illustrative embodiment, a peptidomimetic can be derived as a retro-enantio analog of a peptide. Retro-enantio analogs such as this can be synthesized with commercially available D-amino acids (or analogs thereof) and standard solid- or solution-phase peptide-synthesis techniques, as described, e.g., in WO 00/01720. The final product may be purified by HPLC to yield the pure retro-enantio analog.

In still another illustrative embodiment, trans-olefin derivatives can be made for the subject peptide. Trans-olefin analogs can be synthesized according to the method of Y.K. Shue et al. (1987) Tetrahedron Letters 28:3225 and as described in WO 00/01720. It is further possible to couple pseudodipeptides synthesized by the above method to other pseudodipeptides, to make peptide analogs with several olefinic functionalities in place of amide functionalities.

Still another class of peptidomimetic derivatives include the phosphonate derivatives. The synthesis of such phosphonate derivatives can be adapted from known synthesis schemes. See, for example, Loots et al. in Peptides: Chemistry and Biology, (Escom Science Publishers, Leiden, 1988, p. 118); Petrillo et al. in Peptides: Structure and Function (Proceedings of the 9th American Peptide Symposium, Pierce Chemical Co. Rockland, IL, 1985).

Many other peptidomimetic structures are known in the art and can be readily adapted for use in the subject peptidomimetics. To illustrate, a peptidomimetic may incorporate the 1-azabicyclo[4.3.0]nonane surrogate (see Kim et al. (1997) J. Org. Chem. 62:2847), or an *N*-acyl piperazic acid (see Xi et al. (1998) J. Am. Chem. Soc. 120:80), or a 2-substituted piperazine moiety as a constrained amino acid analogue (see Williams et al. (1996) J. Med. Chem. 39:1345-1348). In still other embodiments, certain amino acid residues can be replaced with aryl and bi-aryl moieties, e.g., monocyclic or bicyclic aromatic or heteroaromatic nucleus, or a biaromatic, aromatic-heteroaromatic, or biheteroaromatic nucleus.

The subject peptidomimetics can be optimized by, e.g., combinatorial synthesis techniques combined with high throughput screening.

Moreover, other examples of mimetopes include, but are not limited to, protein-based compounds, carbohydrate-based compounds, lipid-based compounds, nucleic acid-based compounds, natural organic compounds, synthetically derived organic compounds, anti-idiotypic antibodies and/or catalytic antibodies, or fragments thereof. A mimetope can be obtained by, for example, screening libraries of natural and synthetic compounds for compounds capable of inhibiting cell survival and/or tumor growth. A mimetope can also be obtained, for example, from libraries of natural and synthetic compounds, in particular, chemical or combinatorial libraries (i.e., libraries of compounds that differ in sequence or size but that have the same building blocks). A mimetope can also be obtained by, for example, rational drug design. In a rational drug design procedure, the three-dimensional structure of a compound of the present disclosure can be analyzed by, for example, nuclear magnetic resonance (NMR) or x-ray crystallography. The three-dimensional structure can then be used to predict structures of potential mimetopes by, for example, computer modelling. The predicted mimetope structures can then be produced by, for example, chemical synthesis, recombinant DNA technology, or by isolating a mimetope from a natural source (e.g., plants, animals, bacteria and fungi).

"Peptides, variants and derivatives thereof' or "peptides and analogs thereof" are included in "peptide therapeutics" and is intended to include any of the peptides or modified forms thereof, e.g., peptidomimetics, described herein. Preferred peptide therapeutics decrease cell survival or increase apoptosis. For example, they may decrease cell survival or increase apoptosis by a factor of at least about 2 fold, 5 fold, 10 fold, 30 fold or 100 fold, as determined, e.g., in an assay described herein.

The activity of a Sir or FhuC protein, fragment, or variant thereof may be assayed using an appropriate substrate or binding partner or other reagent suitable to test for the suspected activity as described below.

In another embodiment, the activity of a polypeptide may be determined by assaying for the level of expression of RNA and/or protein molecules. Transcription levels may be determined, for example, using Northern blots, hybridization to an oligonucleotide array or by assaying for the level of a resulting protein product. Translation levels may be determined, for example, using Western blotting or by identifying a detectable signal produced by a protein product (e.g., fluorescence, luminescence, enzymatic activity, etc.). Depending on the particular situation, it may be desirable to detect the level of transcription and/or translation of a single gene or of multiple genes.

Alternatively, it may be desirable to measure the overall rate of DNA replication, transcription and/or translation in a cell. In general this may be accomplished by growing the cell in the presence of a detectable metabolite which is incorporated into the resultant DNA, RNA, or protein product. For example, the rate of DNA synthesis may be determined by growing cells in the presence of BrdU which is incorporated into the newly synthesized DNA. The amount of BrdU may then be determined histochemically using an anti-BrdU antibody.

In other embodiments, polypeptides of the disclosure may be immobilized onto a solid surface, including, microtiter plates, slides, beads, films; etc. The polypeptides of the disclosure may be immobilized onto a "chip" as part of an array. An array, having a plurality of addresses, may comprise one or more polypeptides of the disclosure in one or more of those addresses. In one embodiment, the chip comprises one or more polypeptides of the disclosure as part of an array of polypeptide sequences.

In other embodiments, polypeptides of the disclosure may be immobilized onto a solid surface, including, plates, microtiter plates, slides, beads, particles, spheres, films, strands, precipitates, gels, sheets, tubing, containers, capillaries, pads, slices, etc. The polypeptides of the disclosure may be immobilized onto a "chip" as part of an array. An array, having a plurality of addresses, may comprise one or more polypeptides of the disclosure in one or more of those addresses. In one embodiment, the chip comprises one or more polypeptides of the disclosure as part of an array

### 5. Antibodies and Uses Thereof

To produce antibodies against the Sir and FhuC polypeptides described herein, host animals may be injected with Sir or FhuC polypeptides or with Sir or FhuC peptides. Hosts may be injected with peptides of different lengths encompassing a desired target sequence. For example, peptide antigens that are at least 5, 10, 15, 20, 25, 30, 35, 40, 45, 50, 55, 60, 65, 70, 75, 80, 85, 90, 95, 100, 105, 110, 115, 120, 125, 130, 135, 140, 145 or 150 amino acids may be used. Alternatively, if a portion of a protein defines an epitope, but is too short to be antigenic, it may be conjugated to a carrier molecule in order to produce antibodies. Some suitable carrier molecules include keyhole limpet hemocyanin, Ig sequences, TrpE, and human or bovine serum albumen. Conjugation may be carried out by methods known in the art. One such method is to combine a cysteine residue of the fragments with a cysteine residue on the carrier molecule.

In addition, antibodies to three-dimensional epitopes, i.e., non-linear epitopes, may also be prepared, based on, e.g., crystallographic data of proteins, Antibodies obtained from that injection may be screened against the short antigens of proteins described herein. Antibodies prepared against a Sir or FhuC peptide may be tested for activity against that peptide as well as the full length Sir or FhuC protein. Antibodies may have affinities of at least about 10⁻⁶M, 10⁻⁷M, 10⁻⁸M, 10⁻⁹M, 10⁻¹⁰M, 10⁻¹¹M or 10⁻¹²M or higher toward the Sir or FhuC peptide and/or the full length Sir or FhuC protein described herein.

Suitable cells for the DNA sequences and host cells for antibody expression and secretion can be obtained from a number of sources, including the American Type Culture Collection (*"*Catalogue of Cell Lines and Hybridomas" 5th edition (1985) Rockville, Md., U.S.A.).

Polyclonal and monoclonal antibodies may be produced by methods known in the art. Monoclonal antibodies may be produced by hybridomas prepared using known procedures including the immunological method described by Kohler and Milstein, Nature 1975; 256: 495-7; and Campbell in "Monoclonal Antibody Technology, The Production and Characterization of Rodent and Human Hybridomas" in Burdon et al., Eds. Laboratory Techniques in Biochemistry and Molecular Biology, Volume 13, Elsevier Science Publishers, Amsterdam (1985); as well as by the recombinant DNA method described by Huse et al, Science (1989) 246: 1275-81.

Methods of antibody purification are well known in the art. See, for example, Harlow and Lane (1988) Antibodies: A Laboratory Manual, Cold Spring Harbor Laboratory, N.Y. Purification methods may include salt precipitation (for example, with ammonium sulfate), ion exchange chromatography (for example, on a cationic or anionic exchange column run at neutral pH and eluted with step gradients of increasing ionic strength), gel filtration chromatography (including gel filtration HPLC), and chromatography on affinity resins such as protein A, protein G, hydroxyapatite, and anti-antibody. Antibodies may also be purified on affinity columns according to methods known in the art.

Also disclosed are functional equivalents of antibodies, and include, for example, chimerized, humanized, and single chain antibodies as well as fragments thereof. Methods of producing functional equivalents are disclosed in PCT Application WO 93/21319; European Patent Application No. 239,400; PCT Application WO 89/09622; European Patent Application 388,745; and European Patent Application EP 332,424.

Functional equivalents include polypeptides with amino acid sequences substantially the same as the amino acid sequence of the variable or hypervariable regions of the antibodies of the disclosure, "Substantially the same" amino acid sequence is defined herein as a sequence with at least 70%, preferably at least about 80%, and more preferably at least 90% homology to another amino acid sequence as determined by the FASTA search method in accordance with Pearson and Lipman, Proc Natl Acd Sci USA (1988) 85: 2444-8.

Chimerized antibodies may have constant regions derived substantially or exclusively from human antibody constant regions and variable regions derived substantially or exclusively from the sequence of the variable region from a mammal other than a human. Humanized antibodies may have constant regions and variable regions other than the complement determining regions (CDRs) derived substantially or exclusively from the corresponding human antibody regions and CDRs derived substantially or exclusively from a mammal other than a human.

Suitable mammals other than a human may include any mammal from which monoclonal antibodies may be made. Suitable examples of mammals other than a human may include, for example, a rabbit, rat, mouse, horse, goat, or primate.

Antibodies to Sir proteins and FhuC protein as described herein may be prepared as described above. The antibodies to the Sir and FhuC proteins described herein (whole antibodies or antibody fragments) may be conjugated to a biocompatible material, such as polyethylene glycol molecules (PEG) according to methods well known to persons of skill in the art to increase the antibody's half-life. See for example, U.S. Patent No. 6,468,532. Functionalized PEG polymers are available, for example, from Nektar Therapeutics. Commercially available PEG derivatives include, but are not limited to, amino-PEG, PEG amino acid esters, PEG-hydrazide, PEG-thiol, PEG-succinate, carboxymethylated PEG, PEG-propionic acid, PEG amino acids, PEG succinimidyl succinate, PEG succinimidyl propionate, succinimidyl ester of carboxymethylated PEG, succinimidyl carbonate of PEG, succinimidyl esters of amino acid PEGs, PEG-oxycarbonylimidazole, PEG-nitrophenyl carbonate, PEG tresylate, PEG-glycidyl ether, PEG-aldehyde, PEG vinylsulfone, PEG-maleimide, PEG-orthopyridyl-disulfide, heterofunctional PEGs, PEG vinyl derivatives, PEG silanes, and PEG phospholides. The reaction conditions for coupling these PEG derivatives will vary depending on the polypeptide, the desired degree of PEGylation, and the PEG derivative utilized. Some factors involved in the choice of PEG derivatives include: the desired point of attachment (such as lysine or cysteine R-groups), hydrolytic stability and reactivity of the derivatives, stability, toxicity and antigenicity of the linkage, suitability for analysis, etc.

### 6. Pharmaceutical Compositions

*S. aureus* SirA, SirB, SirC, or FhuC antibodies, antisense nucleic acids, siRNAs, and other antagonists, may be administered by various means, depending on their intended use, as is well known in the art. For example, if such *S. aureus* antagonists compositions are to be administered orally, they may be formulated as tablets, capsules, granules, powders or syrups. Alternatively, formulations of the present disclosure may be administered parenterally as injections (intravenous, intramuscular or subcutaneous), drop infusion preparations or suppositories. For application by the ophthalmic mucous membrane route, compositions of the present disclosure may be formulated as eyedrops or eye ointments. These formulations may be prepared by conventional means, and, if desired, the compositions may be mixed with any conventional additive, such as an excipient, a binder, a disintegrating agent, a lubricant, a corrigent, a solubilizing agent, a suspension aid, an emulsifying agent or a coating agent.

In formulations of the disclosure, wetting agents, emulsifiers and lubricants, such as sodium lauryl sulfate and magnesium stearate, as well as coloring agents, release agents, coating agents, sweetening, flavoring and perfuming agents, preservatives and antioxidants may be present in the formulated agents.

Subject compositions may be suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of composition that may be combined with a carrier material to produce a single dose vary depending upon the subject being treated, and the particular mode of administration.

Methods of preparing these formulations include the step of bringing into association compositions of the present disclosure with the carrier and, optionally, one or more accessory ingredients. In general, the formulations are prepared by uniformly and intimately bringing into association agents with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia), each containing a predetermined amount of a subject composition thereof as an active ingredient. Compositions of the present disclosure may also be administered as a bolus, electuary, or paste.

In solid dosage forms for oral administration (capsules, tablets, pills, dragees, powders, granules and the like), the subject composition is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the subject composition moistened with an inert liquid diluent. Tablets, and other solid dosage forms, such as dragees, capsules, pills and granules, may optionally be scored or prepared with coatings and shells, such as enteric coatings and other coatings well known in the pharmaceutical-formulating art.

Liquid dosage forms for oral administration include pharmaceutically acceptable emulsions, microemulsions, solutions, suspensions, syrups and elixirs. In addition to the subject composition, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Suspensions, in addition to the subject composition, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing a subject composition with one or more suitable nonirritating excipients or carriers comprising, for example, cocoa butter, polyethylene glycol, a suppository wax or a salicylate, and which is solid at room temperature, but liquid at body temperature and, therefore, will melt in the body cavity and release the active agent. Formulations which are suitable for vaginal administration also include pessaries, tampons, creams, gels, pastes, foams or spray formulations containing such carriers as are known in the art to be appropriate.

Dosage forms for transdermal administration of a subject composition includes powders, sprays, ointments, pastes, creams, lotions, gels, solutions, patches and inhalants. The active component may be mixed under sterile conditions with a pharmaceutically acceptable carrier, and with any preservatives, buffers, or propellants which may be required.

The ointments, pastes, creams and gels may contain, in addition to a subject composition, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to a subject composition, excipients such as lactose, talc, silicic acid, aluminum hydroxide, calcium silicates and polyamide powder, or mixtures of these substances. Sprays may additionally contain customary propellants, such as chlorofluorohydrocarbons and volatile unsubstituted hydrocarbons, such as butane and propane.

Compositions of the present disclosure may alternatively be administered by aerosol. This is accomplished by preparing an aqueous aerosol, liposomal preparation or solid particles containing the compound. A non-aqueous (e.g., fluorocarbon propellant) suspension could be used. Sonic nebulizers may be used because they minimize exposing the agent to shear, which may result in degradation of the compounds contained in the subject compositions.

Ordinarily, an aqueous aerosol is made by formulating an aqueous solution or suspension of a subject composition together with conventional pharmaceutically acceptable carriers and stabilizers. The carriers and stabilizers vary with the requirements of the particular subject composition, but typically include non-ionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Pharmaceutical compositions of this disclosure suitable for parenteral administration comprise a subject composition in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity may be maintained, for example, by the use of coating materials, such as lecithin, by the maintenance of the required particle size in the case of dispersions, and by the use of surfactants.

The pharmaceutical compositions described herein may be used to prevent or treat conditions or dieseases resulting from *S. aureus* infections including, but not limited to a furuncle, chronic furunculosis, impetigo, acute osteomyelitis, pneumonia, endocarditis, scalded skin syndrome, toxic shock syndrome, and food poisoning.

### 7. Exemplary Screening Assays for Inhibitors of the SirABC mediated iron transport system of S. aureus

In general, agents or compounds capable of inhibiting pathogenic virulence are identified from large libraries of both natural product or synthetic (or semi-synthetic) extracts or chemical libraries according to methods known in the art. Those skilled in the field of drug discovery and development will understand that the precise source of agents (e.g. test extracts or compounds) is not critical to the screening procedure(s) of the invention. Accordingly, virtually any number of chemical extracts or compounds can be screened using the methods described herein. Examples of such agents, extracts, or compounds include, but are not limited to, plant-, fungal-, prokaryotic- or animal-based extracts, fermentation broths, and synthetic compounds, as well as modification of existing compounds. Numerous methods are also available for generating random or directed synthesis (*e.g*., semi-synthesis or total synthesis) of any number of chemical compounds, including, but not limited to, saccharide-, lipid-, peptide-, and nucleic acid-based compounds. Synthetic compound libraries are commercially available from Brandon Associates (Merrimack, NH) and Aldrich Chemical (Milwaukee, Wis.). Alternatively, libraries of natural compounds in the form of bacterial, fungal, plant, and animal extracts are commercially available from a number of sources, including Biotics (Sussex, UK), Xenova (Slough, UK), Harbor Branch Oceangraphics Institute (Ft Pierce, Fla.), and PharmnaMar, U.S.A. (Cambridge, Mass.). In addition, natural and synthetically produced libraries are produced, if desired, according to methods known in the art, *e.g*., by standard extraction and fractionation methods. Furthermore, if desired, any library or compound is readily modified using standard chemical, physical, or biochemical methods.

In addition, those skilled in the art of drug discovery and development readily understand that methods for dereplication (*e.g*., taxonomic dereplication, biological dereplication, and chemical dereplication, or any combination thereof) or the elimination of replicates or repeats of materials already known for their anti-pathogenic activity should be employed whenever possible.

When a crude extract is found to have an anti-pathogenic or anti-virulence activity, or a binding activity, further fractionation of the positive lead extract is necessary to isolate chemical constituents responsible for the observed effect. Thus, the goal of the extraction, fractionation, and purification process is the careful characterization and identification of a chemical entity within the crude extract having anti-pathogenic activity. Methods of fractionation and purification of such heterogeneous extracts are known in the art. If desired, compounds shown to be useful agents for the treatment of pathogenicity are chemically modified according to methods known in the art.

Potential inhibitors of SirA, SirB, SirC, staphylobactin, or FhuC may include organic molecules, peptides, peptide mimetics, polypeptides, antibodies, antisense RNA, and siRNAs that bind to a nucleic acid sequence or polypeptide of the disclosure and thereby inhibit its activity. Potential antagonists also include small molecules that bind to and occupy the binding site of the polypeptide thereby preventing binding to cellular binding molecules, such that normal biological activity is prevented. Other potential antagonists include antisense molecules.

### 7.1. Interaction Assays

Purified and recombinant SirA, SirB, SirC, staphylobactin and FhuC polypeptides may be used to facilitate the development of assays to screen for agents that modulate the interaction between SirA, SirB, and SirC or between SirA, SirB, SirC, staphylobactin, and/or FhuC. Potential inhibitors of SirA, SirB, SirC, staphylobactin, or FhuC may include small organic molecules, peptides, polypeptides, peptide mimetics, and antibodies that bind to either SirA, SirB, SirC, staphylobactin or FhuC and thereby inhibit its activity.

In an exemplary screening assay, a reaction mixture may be generated to include at least a biologically active portion of either SirA, SirB, SirC, staphylobactin or FhuC polypeptide, agent(s) of interest, and an appropriate interacting molecule. As used herein, the "appropriate interacting molecule" may be SirA, SirB, SirC, staphylobactin or FhuC depending on which polypeptide is used in the screening assay. For example, when SirA is used, an appropriate interacting molecule may be staphylobactin, SirB or SirC. Detection and quantification of interaction of a particular Sir polypeptide with the appropriate interacting molecule provides a means for determining an agent's efficacy at inhibiting interaction between for example, SirA and Sir B. The efficacy of the agent can be assessed by generating dose response curves from data obtained using various concentrations of the test agent. Moreover, a control assay can also be performed to provide a baseline for comparison. In the control assay, interaction of SirA, SirB, SirC, staphylobactin or FhuC polypeptide with the appropriate interacting molecule may be quantitated in the absence of the test agent.

Interaction between SirA, SirB, SirC, staphylobactin or FhuC polypeptide and the appropriate interacting molecule may be detected by a variety of techniques. Inhibition of the formation of complexes can be quantitated using, for example, detectably labeled proteins such as radiolabeled, fluorescently labeled, or enzymatically labeled polypeptides, by immunoassay, or by chromatographic detection.

The measurement of the interaction of a particular Sir or FhuC protein with the appropriate interacting molecule may be observed directly using surface plasmon resonance technology in optical biosensor devices. This method is particularly useful for measuring interactions with larger (>5 kDa) polypeptides and can be adapted to screen for inhibitors of the protein-protein interaction.

Alternatively, it will be desirable to immobilize either SirA, SirB, SirC, staphylobactin or FhuC or the appropriate interacting molecule to facilitate separation of complexes from uncomplexed forms of one or both of the proteins, as well as to accommodate automation of the assay. Binding of SirA to the interacting molecule for example, in the presence and absence of a candidate agent, can be accomplished in any vessel suitable for containing the reactants. Examples include microtitre plates, test tubes, and micro-centrifuge tubes. In one embodiment, a fusion protein can be provided which adds a domain that allows the protein to be bound to a matrix. For example, glutathione-S-transferase/SirA (GST/SirA) fusion proteins can be adsorbed onto glutathione sepharose beads (Sigma Chemical, St. Louis, Mo.) or glutathione derivatized microtitre plates, which are then combined with *e.g.* an ³⁵S-labeled interacting molecule, and the test agent, and the mixture incubated under conditions conducive to complex formation, *e.g*. at physiological conditions for salt and pH, though slightly more stringent conditions may be desired. Following incubation, the beads are washed to remove any unbound label, and the matrix immobilized and radiolabel determined directly (*e.g.* beads placed in scintillant), or in the supernatant after the complexes are subsequently dissociated. Alternatively, the complexes can be dissociated from the matrix, separated by SDS-PAGE, and the level of interacting molecule found in the bead fraction quantitated from the gel using standard electrophoretic techniques.

Other techniques for immobilizing proteins and other molecules on matrices are also available for use in the subject assay. For instance, either SirA, SirB, SirC, staphylobactin, FhuC or the appropriate interacting molecule can be immobilized utilizing conjugation of biotin and streptavidin. For instance, biotinylated SirA, SirB, SirC, staphylobactin or FhuC can be prepared from biotin-NHS (N-hydroxy-succinimide) using techniques well known in the art (*e.g*., biotinylation kit, Pierce Chemicals, Rockford, Ill.), and immobilized in the wells of streptavidin-coated 96 well plates (Pierce Chemical). Alternatively, antibodies reactive with either SirA, SirB, SirC, staphylobactin or FhuC, but which do not interfere with the interaction between the polypeptides and the interacting molecule, can be derivatized to the wells of the plate, and SirA, SirB, SirC, staphylobactin or FhuC may be trapped in the wells by antibody conjugation. As above, preparations of an interacting molecule and a test compound may be incubated in the polypeptide-presenting wells of the plate, and the amount of complex trapped in the well can be quantitated in the presence or absence of a test agent. Exemplary methods for detecting such complexes, in addition to those described above for the GST-immobilized complexes, include immunodetection of complexes using antibodies reactive with the interacting molecule or enzyme-linked assays which rely on detecting an enzymatic activity associated with the interacting molecule.

For example, an enzyme can be chemically conjugated or provided as a fusion protein with the interacting molecule. To illustrate, the interacting molecule can be chemically cross-linked or genetically fused with horseradish peroxidase, and the amount of polypeptide trapped in the complex can be assessed with a chromogenic substrate of the enzyme, *e.g*. 3,3'-diamino-benzadine terahydrochloride or 4-chloro-1-napthol. Likewise, a fusion protein comprising the polypeptide and glutathione-S-transferase can be provided, and complex formation quantitated by detecting the GST activity using 1-chloro-2,4-dinitrobenzene (Habig et al. (1974) J. Biol. Chem. 249:7130).

### 7.2. Iron-transport assays

Alternatively, screening assays may be developed to screen for agents that modulate the iron-transport activity of the Sir polypeptides. Appropriate concentrations of test agents for modulating the iron-transport activity of the Sir proteins can be determined by any method known to one skilled in the art.

In one embodiment, the screening assay may include whole *S. aureus* cells expressing wild type SirA, SirB, SirC or FhuC polypeptides. The ability of a compound to alter the iron transport activity of said polypeptides can be detected by analysis of the cells. For example, antagonists of iron-transport can by detected by scoring for alterations in growth or differentiation (phenotype) of the cell in iron-replete media. The growth of wild-type *S. aureus* strains in the presence of test agent(s) may be compared with the growth of SirA, SirB or FhuC deficient *S. aureus* strains. Each culture may be treated with a test agent from a library of compounds or natural extracts, and monitored for the effect that the particular agent has on the growth on the wild-type and the Sir-deficient strain or FhuC-deficient strain. Bacterial growth may be monitored using a Klett meter. Compounds that specifically interfere with the SirABC iron siderophore transport system will affect only the growth of the wild-type strain.

Alternatively, *S. aureus* cells may be cultured and treated with test agents and then screened for the presence of iron in the cell using atomic absorption spectroscopy techniques (Cox (1994) Meth. Enzymol. 235:315).

Alternatively, inhibition of the iron transport activity may be measured by using radioactively labeled iron. Compounds that interfere with the SirABC iron siderophore transport system will result in a lowered uptake of the radioactively labeled iron. A control assay can also be performed to provide a baseline for comparison. In the control assay, the uptake of radioactively labeled iron in a *S. aureus* cell may be quantitated in the absence of the test compound. Examples of radioactively labeled iron may include ⁵⁹Fe or ⁵⁵Fe.

### 7.3. Expression assays

In a further embodiment, antagonists of the iron transport system may affect the expression of *sirA, sirB, sirC* or *fhuC* nucleic acid or protein. In this screen, *S. aureus* cells may be treated with a compound(s) of interest, and then assayed for the effect of the compound(s) on *sirA, sirB, sirC* or *fhuC* nucleic acid or protein expression.

For example, total RNA can be isolated from *S*. *aureus* cells cultured in the presence or absence of test agents, using any suitable technique such as the single-step guanidinium-thiocyanate-phenol-chloroform method described in Chomczynski et al. (1987) Anal. Biochem. 162:156-159. The expression of *sirA, sirB, sirC* or *fhuC* may then be assayed by any appropriate method such as Northern blot analysis, the polymerase chain reaction (PCR), reverse transcription in combination with the polymerase chain reaction (RT-PCR), and reverse transcription in combination with the ligase chain reaction (RT-LCR).

Northern blot analysis can be performed as described in Harada et al. (1990) Cell 63:303-312. Briefly, total RNA is prepared from *S. aureus* cells cultured in the presence of a test agent. For the Northern blot, the RNA is denatured in an appropriate buffer (such as glyoxal/dimethyl sulfoxide/sodium phosphate buffer), subjected to agarose gel electrophoresis, and transferred onto a nitrocellulose filter. After the RNAs have been linked to the filter by a UV linker, the filter is prehybridized in a solution containing formamide, SSC, Denhardt's solution, denatured salmon sperm, SDS, and sodium phosphate buffer. A *S*. *aureus sirA, sirB, sirC* or *fhuC* DNA sequence may be labeled according to any appropriate method (such as the ³²P-multiprimed DNA labeling system (Amersham)) and used as probe. After hybridization overnight, the filter is washed and exposed to x-ray film. Moreover, a control can also be performed to provide a baseline for comparison. In the control, the expression of *sirA, sirB, sirC* or *fhuC* in *S. aureus* may be quantitated in the absence of the test agent.

Alternatively, the levels of mRNA encoding SirA, SirB, SirC or FhuC polypeptides may also be assayed, for *e.g.,* using the RT-PCR method described in Makino et al. (1990) Technique 2:295-301. Briefly, this method involves adding total RNA isolated from *S*. *aureus* cells cultured in the presence of a test agent, in a reaction mixture containing a RT primer and appropriate buffer. After incubating for primer annealing, the mixture can be supplemented with a RT buffer, dNTPs, DTT, RNase inhibitor and reverse transcriptase. After incubation to achieve reverse transcription of the RNA, the RT products are then subject to PCR using labeled primers. Alternatively, rather than labeling the primers, a labeled dNTP can be included in the PCR reaction mixture. PCR amplification can be performed in a DNA thermal cycler according to conventional techniques. After a suitable number of rounds to achieve amplification, the PCR reaction mixture is electrophoresed on a polyacrylamide gel. After drying the gel, the radioactivity of the appropriate bands may be quantified using an imaging analyzer. RT and PCR reaction ingredients and conditions, reagent and gel concentrations, and labeling methods are well known in the art. Variations on the RT-PCR method will be apparent to the skilled artisan. Other PCR methods that can detect the nucleic acid of the present disclosure can be found in PCR Primer: A Laboratory Manual (Dieffenbach et al. eds., Cold Spring Harbor Lab Press, 1995). A control can also be performed to provide a baseline for comparison. In the control, the expression of *sirA, sirB* or *sirC* in *S*. *aureus* may be quantitated in the absence of the test agent.

Alternatively, the expression of SirA, SirB, SirC or FhuC polypeptides may be quantitated following the treatment of *S. aureus* cells with a test agent using antibody-based methods such as immunoassays. Any suitable immunoassay can be used, including, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

For example, SirA, SirB, SirC, or FhuC polypeptides can be detected in a sample obtained from S. *aureus* cells treated with a test agent, by means of a two-step sandwich assay. In the first step, a capture reagent (e.g., either a SirA, SirB, SirC, or FhuC antibody) is used to capture the specific polypeptide. The capture reagent can optionally be immobilized on a solid phase. In the second step, a directly or indirectly labeled detection reagent is used to detect the captured marker. In one embodiment, the detection reagent is an antibody. The amount of SirA, SirB, SirC, or FhuC polypeptide present in *S*. *aureus* cells treated with a test agent can be calculated by reference to the amount present in untreated *S. aureus* cells.

Suitable enzyme labels include, for example, those from the oxidase group, which catalyze the production of hydrogen peroxide by reacting with substrate. Glucose oxidase is particularly preferred as it has good stability and its substrate (glucose) is readily available. Activity of an oxidase label may be assayed by measuring the concentration of hydrogen peroxide formed by the enzyme-labeled antibody/substrate reaction. Besides enzymes, other suitable labels include radioisotopes, such as iodine (¹²⁵I, ¹²¹I), carbon (¹⁴C), sulphur (³⁵S), tritium (³H).

Examples of suitable fluorescent labels include a fluorescein label, an isothiocyanate label, a rhodamine label, a phycoerythrin label, a phycocyanin label, an allophycocyanin label, an o-phthaldehyde label, and a fluorescamine label.

Examples of suitable enzyme labels include malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholine esterase. Examples of chemiluminescent labels include a luminol label, an isoluminol label, an aromatic acridinium ester label, an imidazole label, an acridinium salt label, an oxalate ester label, a luciferin label, a luciferase label, and an aequorin label.

### Exemplification

The invention, having been generally described, may be more readily understood by reference to the following examples, which are included merely for purposes of illustration of certain aspects and embodiments of the present invention, and are not intended to limit the invention in any way.

### Example 1: Materials and Methods for Examples 2-5

### Media and growth conditions

Bacterial strains, plasmids and oligonucleotides used in Examples 2-5 are listed in Table 1. For routine cloning and protein expression, *E. coli* was grown at 37°C in Luria-Bertani (LB) broth supplemented with erythromycin (300 µg/ml), ampicillin (100 µg/ml), tetracycline (10 µg/ml), chloramphenicol (30 µg/ml) or kanamycin (30 µg/ml), as required. For general manipulations, *S*. *aureus* strains were cultured in tryptic soy broth (TSB) (Difco) containing erythromycin (5 µg/ml), tetracycline (4 µg/ml), kanamycin and neomycin (50 µg/ml) or chloramphenicol (5 µg/ml), as required. For iron-restricted bacterial growth experiments, a Tris minimal succinate (TMS) medium was used, the composition of which has been previously described (Sebulsky et al. (2000) J. Bacteriol. 182:4394-4400). TMS was supplemented with 2'2-dipyridyl, at concentrations as described in the following Examples, to further restrict the concentration of free iron in the media.

### Plasmid and strain constructions

All DNA manipulations and plasmid constructions were performed using standard protocols. The *sirABC* operon was PCR-amplified from the chromosome of *S*. *aureus* 8325-4 using *Pwol* polymerase (Roche Diagnostics) and primers *Sir* upper and *Sir* lower. The resultant 3.8-kb product was cloned into the *SmaI* site of pBC SK+ to create pSirABC.

To interrupt the *sirA* coding region, pSirABC was digested with *NsiI,* filled with T4 DNA polymerase, and ligated to a kanamycin resistance cassette that had been excised as a *StuI*/*SmaI* fragment from pDG782. The *sirA*::Km region was then cloned into *BamHI*/*KpnI*-digested pAUL-A, creating plasmid pMTS12.

The *sirB* coding region was interrupted by insertion of a tetracycline resistance cassette, derived from digesting pDG1513 with *ClaI* (end-polished with Klenow enzyme), into the *StuI* site of *sirB.* The sirB::tet fragment was cloned into *BamHI*/*KpnI-*digested pAUL-A, creating plasmid pSirB::Tet3.

To create strains bearing individual mutations in *sirA* and *sirB,* pMTS12 and pSirB::Tet3 were introduced into *S. aureus* RN4220, followed by transduction via phage 80α, into *S. aureus* RN6390. Transductants were confirmed by restriction analysis. Allelic replacement was accomplished by growing plasmid-containing bacteria at 30°C for three hours followed by a shift in growth temperature to 43 °C for a further four hours. Double crossover events were screened for by resistance to kanamycin (for *sirA*::Km mutation) or tetracycline (for *sirB*::Tet mutation) with a concomitant loss of erythromycin resistance in both cases. PCR and Southern blot analyses were used to verify the insertion of the antibiotic resistance cassettes into *sirA* and *sirB.* The resulting mutant strains were designated H306 (RN6390 *sirA*::Km) and H474 (RN6390 *sirB*::Tet)*.* Transduction was used to mobilize the mutations into different genetic backgrounds, such as *S. aureus* Newman.

For complementation of the *sirA*::Km mutation, the entire *sirABC* operon was excised from pSirABC (using *KpnI* and *BamHI*) and cloned into pAW8 to create plasmid pSED44. For complementation of the *sirB*::Tet mutation, the *sirB* coding region was PCR-amplified from the *S*. *aureus* RN6390 chromosome using the primers *SirB* Comp 5' and *SirB* Comp 3', followed by digestion with *KpnI* and *SacI* for directional cloning into pALC2073, to create plasmid pSED43. Complementing vectors were electroporated into *S*. *aureus* RN4220 and transduced into mutant strains using bacteriophage 80α using methodologies previously described (Sebulsky et al. (2000) J. Bacteriol. 182:4394-4400).

### RT-PCR

Total RNA for use in RT-PCR reactions was isolated from bacterial cultures in late logarithmic phase using TRIzol® Reagent (Invitrogen). RNA samples were treated with DNaseI for 15 minutes at room temperature prior to the RT-PCR reactions. The SuperScript™ One-Step RT-PCR with Platinum® *Taq* kit (Invitrogen) kit was used according to manufacturer's instructions. 500 ng of total RNA was reverse transcribed using primers SirB Internal 5' and SirB Internal 3' to amplify a 399-bp fragment internal to the *sirB* coding region. As an internal control, a 483-bp fragment of *gap* (encodes glyceraldehyde-3-phosphate dehydrogenase) was amplified using the Gapdh 5' and Gapdh3' oligonucleotide primers.

### Bacterial growth curves

*S. aureus* cultures were pre-grown overnight in TMS. Cells were washed before approximately 10⁷ colony forming units of each strain were inoculated into fresh TMS media containing 250 µM 2,2'-dipyridyl (Sigma) with, or without, 50 µM FeCl₃. Bacterial growth was monitored using a Klett meter until late stationary phase was reached.

### Siderophore plate bioassays

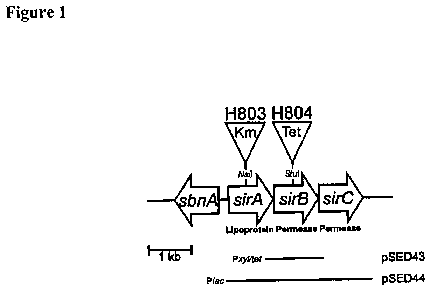

Siderophore plate bioassays were performed essentially as previously described (Sebulsky et al. (2000) J. Bacteriol. 182:4394-4400) with the following modifications: TMS-agar was cooled to 45°C before the addition of 105 cfu/ml of each strain to be tested. 2,2'-dipyridyl was added at a concentration of 550 µM for plates containing *S*. *aureus* Newman and Newman containing vehicle controls (*e.g*. pAW8 and pALC2073) or 400 µM 2,2'-dipyridyl for strains H803 (Newman *sirA*::Km) and H804 (Newman *sirB::Tet)* (see Figure 1) with or without plasmids. Staphylobactin siderophore was isolated from RN6390 as previously described (Dale et al. (2004) Infect. Immun. 72:29-37). Briefly, *S*. *aureus* strains were vigorously shaken in TMS for 48 hours at 37°C. Culture supernatants were recovered by centrifugation in 100% methanol to 1/10 of the volume of the original culture supernatant and passed through Whatman No. 1 filter paper to remove particulate material. Rotary evaporation was used to reduce the volume before application to an LH-20 column (Amersham Biosciences). Fractions were collected, and those testing positive with chrome azurol *S* (CAS) shuttle solution and for biological activity in siderophore plate bioassays were dried, resuspended in water, and examined by high-performance liquid chromatography (HPLC). Analytical reversed-phase HPLC was used for final purification of siderophore. The column utilized was a Waters ODS2 SPherisorb column (4.6 by 150 mm). Trifluoroacetic acid (0.1 %) in water represented solvent A whereas 0.1% trifluoroacetic acid in acetonitrile was used as solvent B. The chromatographic method used was as follows: at a flow rate of 0.75 ml/min, 6% B for 3.5 min., followed by a gradient of 6 to 60% B over 20 min. Staphylobactin was detected at 210 nm and had a retention time of approximately 17 min. Staphylobactin was collected, dried, and rechromatographed to check for purity. The purity of Staphylobactin siderophore was confirmed by HPLC analysis. Aerobactin was purchased from EMC microcollections and used at a concentration of 1 µg/ml as a control in all bioassays.

### ⁵⁵Fe transport assays

*S. aureus* strains were grown to late-logarithmic phase in TMS containing 100 µM 2,2'-dipyridyl with, or without, 50 µM FeCl₃. Cells were washed twice with TMS over a 0.45 µm filter (Gelman) and normalized to an OD600 = 1.2. Twenty minutes prior to 160 the assay, ⁵⁵FeCl₃ (75 µM) was mixed with approximately 220 µM staphylobactin (calculated from DESFERAL® equivalents) in the presence of 2 µM nitrilotriacetic acid (NTA) and allowed to equilibrate at room temperature. Uptake was initiated by adding 10 µl of the ⁵⁵Fe-staphylobactin mixture to 1-ml volumes of cells. At various time points, 200 µl of cells were removed and washed twice with 100 mM LiCl2 over a 0.45 µm membrane. Membranes were dried and counted in CYTOSCINT® fluid using the tritium channel of a Beckman LS 6500 scintillation system. In some experiments, *S. aureus* were treated with 10 mM potassium cyanide (KCN) at room temperature for 20 minutes prior to addition of ⁵⁵Fe mixture. Data presented are pmoles of ⁵⁵Fe transported normalized to the total protein content of the cells (+/- standard deviation) as determined by Bradford assays.

### Transcriptional lacZ fusions and β-gatactosidase assays

The creation of lacZ transcriptional fusions to *sbnA, sbnF* and *sbnI* has been previously described (Dale et al. (2004) Infect. Immun. 72:29-37). Internal fragments of individual genes were cloned into the multiple cloning site of pMUTIN4 (Vagner et al., (1998) Microbiology 144:3097-3104), a vector that does not replicate in Gram-positive bacteria. These fusions were transduced into H306 and H474 genetic backgrounds and the presence of mutations and gene fusions were confirmed by PCR. The construction of a sbnH::lacZ transcriptional fusion has also been previously described (Dale et al. (2004) Infect. Immun. 72:29-37). This fusion was transduced into Newman, H803 and H804 genetic backgrounds and the presence of the gene fusion was confirmed by PCR.

For quantitation of β-galactosidase expression from *S. aureus,* cells were grown to an optical density at 600 nm of 0.8 in TMS supplemented with 100 µM 2,2'-dipyridyl and assayed as previously described (Dale et al. (2004) Infect. Immun. 72:29-37). Briefly, cultures were lysed in a solution containing 10 mM potassium phosphate buffer (pH 7.5), 15 mM EDTA, 1 % Triton X-100, and 10 µg of lysostaphin at 37C. After centrifugation of cell debris, 5 µl of supernatant was assayed for β-galactosidase activity using the Galacto-Light Plus Chemiluminescent reporter gene kit (Tropix) in a Berthold luminometer. The background was set at 50 relative light units/s and the data presented are mean relative light units per second for three independent samples ± standard error.

### Purification of SirA and generation of anti-SirA antisera

We expressed SirA, minus the signal peptide, in *E. coli* ER2566 by cloning *sirA,* amplified from the genome of *S. aureus* using primers pSirA (BamHI) and pSirA (EcoRI), into pGEX-2T-TEV digested with *EcoRI* and *BamHI.* This construct, named pSirA, was grown to mid-log phase before being induced with 0.5 mM IPTG for four hours. Cells were lysed using a French Press and the lysate was centrifuged at 40,000 rpm to pellet cell debris. The supernatant was applied to a GSTrap (Amersham Biosciences) column equilibrated with PBS and the GST-SirA fusion protein was eluted with 10 mM reduced glutathione in 50 mM Tris-Cl, pH 8.0. SirA was cleaved from GST by incubation with tobacco etch virus protease for 3 hours at room temperature and dialyzed overnight at 4°C against 50 mM Tris-Cl, pH 8.0. SirA was further purified using a Mono S column (Amersham Biosciences) equilibrated with sodium phosphate buffer, pH 7.0 and the protein was eluted in sodium phosphate buffer containing 1M NaCl. The purity of SirA was confirmed by SDS-PAGE.

Antibodies recognizing SirA were generated in New Zealand white rabbits (Charles River) inoculated subcutaneously with 500 µg of SirA emulsified in 100 µl Freund's complete adjuvant. On days 14 and 28, rabbits received booster injections of 100 µg of SirA emulsified in Freund's incomplete adjuvant. Rabbits were sacrificed 10 days following the second boost. Antisera were adsorbed against H306 cell lysates and used at a 1:2000 dilution for Western blots.

### Example 2: Expression of SirA is iron-regulated via Fur

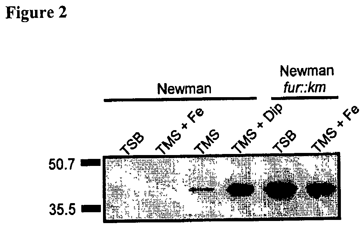

Although SirA expression was undetectable in S. *aureus* Newman cultured in iron-replete media (either TSB or TMS containing 50 µM FeCl₃), its expression was readily detectable during upon growth under conditions of iron restriction (Figure 2). Expression levels increased as the level of iron restriction increased (*i.e.* when 2,2'-dipyridyl was added to TMS) (Figure 2). These findings are in agreement with previous studies which used S. *aureus* 8325-4 (Heinrichs et al. (1999) J. Bacteriol. 181:1436-1443). We have further demonstrated that SirA expression was controlled by the activity of the Fur protein in S. *aureus,* since SirA expression was no longer iron-regulated in a Fur-deficient background (Figure 2). This finding is in agreement with the predicted presence of a consensus Fur box upstream of *sirA* (Dale et al. (2004) Infect. Immun. 72:29-37; Heinrichs et al. (1999) J. Bacteriol. 181:1436-1443).

### Example 3: In the absence of sirB and sirC, the sirA gene product is toxic to E. coli

Many unsuccessful attempts, using different vectors and promoter systems (data not shown), were made to clone the *sirA* coding region for complementation of H803. These unsuccessful results indicated that 'leaky' expression of even small quantities of this lipoprotein was lethal to *E. coli.* The problems encountered with the cloning of *sirA,* appear not to be due to the soluble or amphiphilic regions of the protein since, for the generation of anti-SirA antisera, *sirA* lacking the signal peptide was successfully cloned into an *E. coli* expression vector and large quantities of soluble SirA was produced. These results lend support to the idea that the problems encountered with cloning *sirA* may be due to improper processing of the lipoprotein in *E. coli.*

Interestingly, however, the apparent toxic effect of the SirA lipoprotein on *E. coli* occurred only when attempts were made to clone the *sirA* gene on its own and not when *sirB* and *sirC* were included in the cloned DNA. Indeed, the *sirABC* genes were successfully cloned as a unit on plasmid pSirABC (Table 1) and this plasmid expressed large quantities of SirA in *E. coli.* This result suggests that the transmembrane components of the transporter, components that would presumably interact with SirA at the membrane, may help stabilize the lipoprotein in the membrane.

### Example 4: SirA and SirB are involved in iron acquisition

The Sir polypeptides share similarity with the membrane components of ABC transporters (SirB and SirC) and ligand binding proteins (SirA) and, more specifically, with transporters involved in the acquisition of iron. To address the potential role of *sirABC* in iron acquisition, we used kanamycin and tetracycline resistance cassettes to insertionally-inactivate the coding regions of *sirA* and *sirB,* respectively, in *S*. *aureus* RN6390. Mutations from each of these strains, designated H306 (*S*. *aureus* RN6390 *sirA*::Km) and H474 (*S*. *aureus* RN6390 *sirB*::Tet)*,* were transduced into *S. aureus* Newman to create strains H803 (*sirA*::Km) and H804 (*sirB::Tet*)*.* While SirA was undetectable in H803 (*sirA*::Km), the H804 (*sirB*::Tet) mutant expressed wildtype levels of SirA. A faintly reactive band that migrated faster than SirA is visible in cell extracts of H803. This band is likely due to cross-reactivity with another protein due to the polyclonal antisera that was used. This protein band is likely masked by the high-level expression of the SirA protein in the other samples tested (i.e., SirA expression was detected in E.coli (pSED44) and H686 (*sbnE*::Km))*,* since it is visible when S. *aureus* Newman was grown in iron-replete conditions (Figure 2).

In previous studies, sensitivity to streptonigrin has been used as a method to demonstrate the loss or perturbation of iron import in *S*. *aureus* (Sebulsky et al. (2000) J. Bacteriol. 182:4394-4400). Streptonigrin is toxic to cells in the presence of intracellular iron and, therefore, cells importing iron are generally more sensitive to the toxic affects of this drug versus mutants debilitated in iron import (Yeowell et al. (1982) Antimicrob. Agents Chemother. 22:961-968). The minimum inhibitory concentration (MIC) of streptonigrin was approximately 4-fold lower for *S. aureus* Newman grown in TMS than for either *S*. *aureus* H803 or H804 grown in the same media (see Table 2). Different susceptibilities to streptonigrin were overcome by inclusion of DESFERAL1®, an iron-chelating agent, into the growth media, indicating that this siderophore was used equally well by parent and mutants. These data indicated that SirA and SirB were likely involved in the transport of iron into the cell. As further evidence in this regard, the MIC of 2,2'-dipyridyl (a non-metabolizable iron chelator) for *S*. *aureus* Newman was demonstrated to be 4-fold higher than for either H803 or H804 (Table 2).

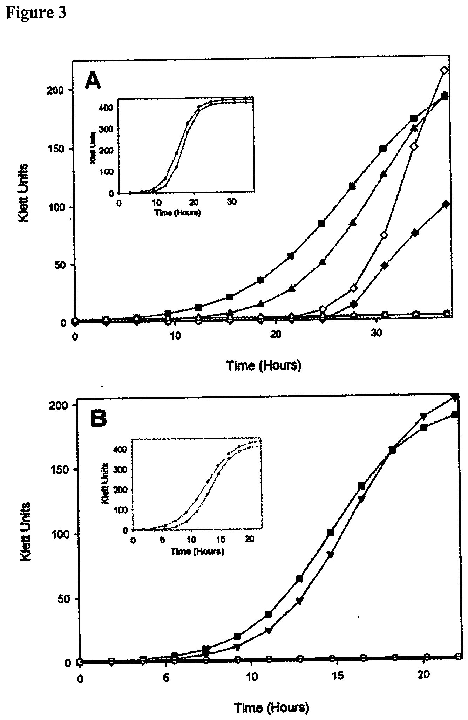

Growth of wild-type Newman and derivatives was followed over a 24-36 hour time period in order to identify deficiencies in growth rate that would correlate with the loss of either *sirA* or *sirB* function. In iron-replete growth media, the growth of H803 (Newman *sirA*::Km) was unaltered in comparison to Newman (Figure 3A, inset). H803, however, showed a drastic growth deficiency compared to Newman in iron-restricted growth media (Figure 3A). Introduction of plasmid pSED44 (contains the *sirABC* operon expressed from Plac; Figure 1) into H803 corrected the growth deficiency of this strain in iron-restricted growth media. The P*lac* promoter in the pAW8 vector expresses large quantities of SirA from the pSED44 construct in *E. coli* but extremely little SirA in *S*. *aureus,* even in the presence of 1 mM IPTG (data not shown), indicating that Plac is functional but extremely inefficient in *S*. *aureus.* However, even this small amount of SirA was capable of complementing the mutation in H803. The addition of 1 mM IPTG to the iron-deficient growth media did enhance the complementation (Figure 3). Introduction of vehicle alone into either Newman or H803 did not affect their growth rate (Figure 3).

Since we were unable to complement the *sirA::Km* mutation with the *sirA* coding region alone (see below), we had to rule out the possibility of polarity of the *sirA::Km* mutation on expression of downstream *sir* genes. RT-PCR experiments demonstrated that the *sirA::Km* mutation had no effect on expression of *sirB* (data not shown), while also confirming that expression of *sirB* transcript was regulated by iron concentrations in the growth medium. Further, sirB was not detected in H804, the strain containing the *sirB*::*Tet* mutation, grown under iron starvation conditions since the Tet cassette disrupts the region amplified in the PCR. In these experiments, total RNA (500 ng) was reverse transcribed and the cDNA to *sirB* and *gap* were amplified as described in Example 1.

Similar to H803, the growth of H804 (Newman *sirB*::Tet) in iron-replete media was unaltered in comparison to wild-type Newman (Figure 3B, inset). However, as with H803, the growth of H804 was severely impaired in iron-deficient growth media compared to *S*. *aureus* Newman (Figure 3B). This growth deficiency of H804 was alleviated with the introduction of pSED43 into the strain (Figure 3B); pSED43 expresses *sirB* from a xyl/tet promoter (Figure 1). The xyl/tet promoter was found to be quite leaky in *S. aureus* (data not shown) and therefore it was unnecessary to incorporate inducer (anhydrotetracycline) into the growth media in these experiments to see full restoration of wildtype phenotype.

### Example 5: Mutation of SirA and SirB results in S. aureus defective in staphylobactin transport but not staphylobactin biosynthesis

Staphylobactin was isolated from *S. aureus* RN6390 using previously described techniques (Dale et al. (2004) Infect. Immun. 72:29-37). Purified staphylobactin was used to assess growth promotion in siderophore plate bioassays. While staphylobactin readily promoted the growth of *S. aureus* Newman, RN6390 and H287 (*fhuG*::*Tn917*) in siderophore plate bioassays, no staphylobactin-mediated growth promotion was observed for H306 (RN6390 *sirA*::Km), H474 (RN6390 *sirB::Tet*)*,* H803 (Newman *sirA*::Km) or H804 (Newman *sirB::Tet),* indicating that both SirA and SirB are essential for staphylobactin-mediated iron transport. To confirm these results, purified staphylobactin was incubated with ⁵⁵FeCl₃ and transport assays were performed with *S. aureus* Newman and H803. While significant transport of ⁵⁵Fe-staphylobactin was observed in *S. aureus* Newman, virtually no transport occurred in Newman pre-grown in TMS containing FeCl₃, Newman treated with 10 mM KCN or in H803 (data not shown). Together, these results confirm that staphylobactin transport is an iron-regulated process that requires the function of at least SirA and SirB, and suggest that this transport is an ATP consuming reaction. Growth promotion by aerobactin, DESFERAL® and ferric-citrate was unaffected in *sirA* and *sirB* mutants and growth in the presence of staphylobactin was restored in the complemented *sirA::Km* and *sirB::Tet* mutants (data not shown).

At least in one instance in *S*. *aureus,* more than one gene encoding the lipoprotein (or binding protein) component of a transport system is found in the genome. Indeed, the iron-hydroxamate uptake system in many strains of *S*. *aureus* is comprised of single copies of genes encoding the ABC-transporter components but two genes that encode a binding protein component (*e.g*., fhuD1 and fhuD2) (Sebulsky and Heinrichs (2001) J. Bacteriol. 183:4394-4400; Sebulsky et al. (2003) J. Biol. Chem. 278:49890-900). In strains containing both fhuD1 and fhuD2, mutation of one of the genes leads to a phenotype that is either wildtype or very close to wildtype for iron-hydroxamate uptake (Sebulsky and Heinrichs (2001) J. Bacteriol. 183:4394-4400). Given that both the sirA::Km and sirB::Tet mutations lead to equivalent phenotypes, we conclude that there is only one gene encoding the binding protein (*i.e.,* SirA) component and one copy of genes (*i.e.,* sirB and sirC) encoding the membrane permease for this transport system.

Given that the functions of proteins expressed from the sbn operon and the sir operon are associated (i.e., biosynthesis and import of staphylobactin), we wished to determine whether there were any effects on their expression as a function of mutations in the operons. Mutation of sbnE results in the loss of staphylobactin synthesis (Dale et al. (2004) Infect. Immun. 72:29-37), however, we showed that loss of sbnE function and, therefore, biosynthesis of staphylobactin had no major effect on the expression of SirA (data not shown). In corollary experiments, we investigated whether loss of sirA or sirB resulted in loss of, or decreased, staphylobactin production. We observed that H803, grown in moderately iron-restricted media, produced significant amounts of staphylobactin both by analytical HPLC and ESI-mass spectrometry (data not shown). To investigate this phenomenon further, we transduced a transcriptional lacZ-sbnH fusion into Newman, H803 and H804. We observed a significant increase in transcription of the sbnH gene in the H803 and H804 genetic backgrounds compared to wildtype Newman (Table 3). No transcription of β-galactosidase activity was observed when strains containing the fusion were grown in iron-replete conditions (data not shown). These results suggest that staphylobactin biosynthesis may be enhanced in strains deficient in the ability to transport this siderophore, presumably in response to an elevated iron starvation status.

### Example 6: Material and Methods for Examples 7-12

### Bacterial strains, plasmids, and growth conditions

Bacterial strains and plasmids used in Examples 7-12 are described in Table 4. *E. coli* was grown in Luria-Bertani broth (Difco). For experiments not directly involved in the analysis of iron uptake, *S*. *aureus* was grown in tryptic soy broth (Difco). Tris-minimal succinate (TMS) was prepared as described (Sebulsky et al. (2004) J. Biol. Chem. 279:53152-9) and used as an iron-limited minimal media. To further restrict the level of free iron in TMS, the iron chelating compounds 2,2'-dipyridyl and ethylene diamine-di(*o-*hydroxyphenol acetic acid) (EDDHA) were added as indicated in the text. Where necessary, ampicillin (100 µg/ml) or kanamycin (30 µg/ml) was incorporated into the media for the growth of *E. coli* strains. For *S*. *aureus,* chloramphenicol (5 µg/ml), kanamycin (50 µg/ml), neomycin (50 µg/ml), erythromycin (3 µg/ml), and lincomycin (20 µg/ml) were incorporated into growth media as required. Solid media were obtained by the addition of 1.5% (w/v) Bacto agar (Difco). All bacterial growth was conducted at 37°C unless otherwise stated. Iron-free water for preparation of growth media and solutions was obtained by passage through a Milli-Q water filtration system (Millipore Corp.).

### Recombinant DNA methodology

Standard DNA manipulations were performed essentially as described by Sambrook *et al.* (Sambrook et al. (1989) Molecular cloning: A Laboratory Manual, 2nd ed. Cold Spring Harbor Laboratory Press, Cold Spring Harbor). Restriction endonucleases and DNA-modifying enzymes were purchased from Roche Diagnostics (Laval, Quebec, Canada), New England Biolabs (Mississauga, Ontario, Canada), Life Technologies Inc. (Burlington, Ontario, Canada) and MBI Fermentas (Flamborough, Ontario, Canada). Plasmid DNA was purified using QIAprep plasmid spin columns (QIAgen Inc., Santa Clarita, California) as described by the manufacturer. For plasmid isolation from *S. aureus,* lysostaphin (50 µg/mL) was added to buffer P1. Chromosomal DNA from *S. aureus* was isolated using the InstaGene™ Matrix (Bio-Rad) as described by the manufacturer. Polymerase chain reactions were performed using either *Pwo*I or *Taq* DNA polymerase (Roche Diagnostics).

### Construction of a ΔfhuCBG::ermB mutant

To create a deletion of the *fhuCBG* operon in the chromosome of RN6390, two regions of DNA flanking the operon were amplified from the RN6390 chromosome by PCR. Primers *fhuC* upstream sense (5'-TTGAATTCAATACCTCGATGTAAGCACG-3') and *fhuC* upstream antisense (5'-TTGGATCCACGATTCATAATTTCCCTAC-3') were used to amplify a 709-bp fragment upstream of and including the first 9-bp of the *fhuC* open reading frame, and primers *fhuG* downstream sense (5'-TTGGATCCAACGAAAAATGTATAGTGTC-3') and *fhuG* downstream antisense (5'-TTTCTAGACGGCAAGCTTATGAACAAAC-3') were used to amplify a 718-bp fragment downstream of *fhuG* including the final 13-bp of the *fhuG* open reading frame.

The *fhuC* upstream fragment was digested with EcoR1 and *Bam*HI (recognition sites underlined in primer sequences) and the *fhuG* downstream fragment was digested with *Bam*HI and *Xba*I (recognition sites underlined in primer sequences) and these two fragments were cloned together into pUC19 digested with *Eco*RI and *Xba*I*.* The resulting construct was digested with *Bam*HI and a 1.6-kb *Bam*HI fragment from pDG646, carrying the *ermB* gene, was inserted. The resulting plasmid construct was digested with *Eco*RI and *Xba*I and a 3027-bp fragment harbouring *ermB* between the *fhuC* upstream and *fhuG* downstream fragments was ligated into pAUL-A Km digested with *Eco*RI and *Xba*I to create the plasmid *p*Δ*fhuCBG.*

Plasmid pΔ*fhuCBG* was introduced into *S*. *aureus* RN4220 by electroporation and colonies resistant to kanamycin and neomycin were selected after growth at 30°C. Kanamycin resistant clones were subjected to a temperature shift to 42°C to select for plasmid integration in the chromosome. Bacteria that were resistant to erythromycin and lincomycin but sensitive to kanamycin and neomycin were selected. The Δ*fhuCBG::ermB* mutation was confirmed by PCR and the mutation was subsequently transduced to the RN6390 and Newman backgrounds to create strains H1071 and H1074, respectively.

### Construction of complementing plasmids

In order to complement the *ΔfhuCBG::ermB* mutation, pMTS20 (Sebulsky et al. (2000) J. Bacteriol. 182:4394-4400) was digested with *Bam*HI and the resulting 3.7-kb fragment harbouring the *fhuCBG* operon with approximately 400-bp of upstream DNA (encompassing the Fur box and promoter sequences) was ligated into the *Bam*HI site of pLI50 to create pFhuCBG. To create pFhuC, primers *fhuCBG*2 (5'-TTTGGATCCACAAGTTTCAAAAGCAAAGC-3') and *fhuc* antisense (5'-TTGGATCCATTTGTCATGTTAATTGTCC-3') were used to amplify a 1.2-kb region containing the *fhuC* coding region plus the same 400-bp upstream region as in pFhuCBG, and the resulting PCR product cloned into the *Bam*HI site of pLI50.

### Siderophores

Ferrichrome was purchased from Sigma (Mississauga, Ontario, Canada), desferrioxamine B, used as DESFERAL® (Novartis), was obtained from the London Health Sciences Centre (London, Ontario), and enterobactin was purchased from EMC Microcollections GmbH (Tübingen, Germany). Staphylobactin was prepared as previously described (Dale et al. (2004) J. Bacteriol. 186:8356-62).

### Bioassays

Siderophore plate bioassays were performed essentially as described (Sebulsky et al. (2000) J. Bacteriol. 182:4394-4400). Briefly, 10⁴ cells/mL were added to molten TMS agar containing 25 µM EDDHA as an iron chelating agent. Ten microlitres of iron sources to be tested (DESFERAL® (50 µM), ferrichrome (50 µM), enterobactin (500 µM), staphylobactin (50 µM DESFERAL® equivalents), hemin (250 |ig/mL), hemoglobin (2 mg/mL), FeCl₃ (50 mM), or ferric citrate (5 mM)) were added to sterile 6 mm-diameter paper disks and placed on the surface of the plates. Growth promotion, as measured by the diameter of the growth halo around each disk, was determined after 48 h incubation, except for heme and hemoglobin, which were measured after 72 hours.

### Determination of minimal inhibitory concentration of 2,2'-dipyridyl for S. aureus strains

Strains to be tested were pre-grown in TMS and cells were washed in fresh TMS prior to assay. 2,2'-dipyridyl was added to TMS and serially diluted to give a range from 1 mM to 32 µM. Following serial dilution, 5 x 10⁴ CFUs were added to each 5-mL culture and growth was recorded after 24 hours incubation.

### Bacterial growth curves

Strains were pre-grown overnight in TMS. Cells were washed with TMS and 5 x 10⁶ CFUs were added to 50 mL of fresh TMS or TMS supplemented with 50 µM 2,2'-dipyridyl in acid-washed flasks. Where necessary, 50 µM FeCl₃ was added to the media to create iron-replete conditions. Bacterial growth was monitored by measuring the optical density of the culture at 600 nm (OD₆₀₀) until stationary phase was reached.

### ⁵⁵Fe Transport Assays

Siderophore uptake was measured as previously described (Dale et al. (2004) J. Bacteriol. 186:8356-62) with the following modifications: all strains were grown overnight in TMS containing 50 µM 2,2'-dipyridyl and appropriate antibiotics, with the exception of RN6390 Δ*fhuCBG::ermB* (H1071), which was grown in TMS alone. Cells were washed three times in TMS and normalized to an optical density at 600 nm of 2.0. Siderophores (DESFERAL® and staphylobactin, -200 µM each) were mixed with ⁵⁵FeCl₃ (75 µM) in the presence of 4 µM nitrilotriacetic acid and the mixtures were equilibrated at room temperature for 30 minutes. Iron uptake was initiated by adding 10 µl of each ⁵⁵Fe-siderophore mixture to 1 mL of cells. At various time points, 200 µL of cells were removed and washed twice with 100 mM LiCl over a 0.45 µM pore-size membrane (Pall Gelman). Dried membranes were counted in CytoScint® fluid using the tritium channel of a Beckman LS-6500 scintillation system. In some experiments, bacteria were exposed to 10 mM potassium cyanide for 15 minutes at room temperature prior to initiating uptake with the ⁵⁵Fe-siderophore mixtures. The data presented are the picomoles of ⁵⁵Fe transported normalized to the optical density of the cultures.

### Mouse Kidney Abscess Model

Female Swiss-Webster mice (25g each) were purchased from Charles River Laboratories Canada Inc., and housed in microisolator cages. *S. aureus* Newman and Newman Δ*fhuCBG*::*ermB* (H1074), were grown overnight in TSB, washed three times with sterile saline and suspensions of 1 x 10⁸ cfu/mL were made in sterile saline. One hundred microliters of the cell suspensions were administered intravenously via the tail vein. The number of viable bacteria injected was confirmed by plating serial dilutions of the inocula on TSB-agar. Throughout the course of the experiment, the mice were subjectively scored on their grooming habits and locomotory function. A score of 1 indicated normal function, whereas higher scores indicated altered function (see Results below). On day 7, the mice were euthanized and the kidneys were aseptically removed and homogenized in sterile PBS + 0.1% Triton X-100 using a PowerGen 700 Homogenizer. Homogenates were serially diluted and plated on TSB-agar to enumerate recovered bacteria. Data presented are the log CFU recovered per mouse. The significance of the percentage of kidneys exhibiting visible abscesses in each group was determined using the Z and Fisher's Exact tests.

### Computer analyses

DNA sequence analysis and PCR oligonucleotide primer design were performed using the Vector NTI Suite 7 software package (Informax, Inc.). Microsoft Excel and SigmaPlot (SPSS Inc.) were used for data analysis and graphing applications.

### Example 7: Construction of S. aureus ΔfhuCBG::ermB mutant

The iron-regulated *fhuCBG* operon was previously identified by our laboratory (Sebulsky et al. (2000) J. Bacteriol. 182:4394-4400) and by Xiong *et al.* (Xiong et al. (2000) Microbiology 146:659-668), and was shown to be necessary for transport of iron(III)-hydroxamates in *S. aureus* since mutations in either *fhuB* or *fhuG* eliminated transport (Cabrera et al. (2001) Appl. Environ. Microbiol. 67:1001-3; Sebulsky et al. (2000) J. Bacteriol. 182:4394-4400). The operon is present in all sequenced *S. aureus* genomes (Sebulsky et al. (2004) J. Biol. Chem. 279:53152-9). As part of our studies to elucidate the mechanism of iron(III)-siderophore transport in *S*. *aureus* using the *fhu* system as a model, we constructed a *S*. *aureus* strain, in the RN4220 genetic background, H1068, that contained a *fhuCBG* operon deletion (see Figure 4). The mutation was mobilized by phage transduction to the RN6390 and Newman genetic backgrounds, creating strains H1071 and H1074, respectively.

### Example 8: A ΔfhuCBG mutant is unable to utilize iron(III)-hydroxamates

In agreement with previous results indicating that loss of either *fhuG o*r *fhuB* result in a complete inability to utilize any iron(III)-hydroxamate complexes for iron-restricted growth, plate bioassays showed that H1071 was unable to utilize DESFERAL®, ferrichrome, coprogen, and aerobactin. H1071 was able to utilize all aforementioned hydroxamate siderophores for iron acquisition in siderophore plate bioassays when the strain was provided with the *fhuCBG* operon *in trans,* on plasmid pFhuCBG (data not shown).

### Example 9: Mutation of fhuC, but not other fhu genes, in S. aureus, yields an iron-restricted growth defect

Previously unpublished results from our laboratory indicated that *fhu* mutations in S. *aureus,* for example RN6390 *fhuG*::Tn*917* (H287) and RN6390 *fhuD1*::Km *fhuD2*::Tet (H431), did not have an obvious growth defect in iron-deficient media, suggesting that hydroxamate siderophores are not produced by *S*. *aureus* in response to iron starvation; in agreement, *S*. *aureus* culture supernatants test negative in the Czaky test (Payne (1994) Detection, isolation, and characterization of siderophores, p. 329-344. In V. L. Clark and P. M. Bavoil (ed.), Methods in Enzymology, vol. 235. Academic Press, Inc., San Diego, CA) for hydroxamates (data not shown). Surprisingly, however, we observed that the growth of H1071 was significantly retarded compared to wildtype RN6390, H287 (RN6390 *fhuG*) and H431 (RN6390 *fhuD1 fhuD2*) in iron-deficient TMS media (data not shown) and even more so in TMS containing 50 µM 2,2'-dipyridyl, a non-metabolizable iron chelator (data not shown). Addition of 50 µM ferric chloride to TMS restored growth of H1071 to wild-type levels demonstrating that the impaired growth was due solely to the level of iron available to the bacteria (data not shown). The iron-restricted growth defect demonstrated by H1071 could be complemented by introduction of a plasmid carrying the operon (pFhuCBG). However, more surprising was the observation that *fhu*C alone *in trans,* present on plasmid pFhuC, complemented the iron-restricted growth deficiency of strain H1071, indicating that the growth defect of H1071 was as a result of the inability to express *fhuC.* ⁵⁵Fe-DESFERAL® uptake assays were performed and showed that H1071 was incapable of transporting ⁵⁵Fe-DESFERAL® (data not shown), corroborating bioassay results (see above). Of note, however, was the observation that although pFhuC could complement the growth deficiency of H1071, it could not restore the ability of H1071 to transport ⁵⁵Fe-DESFERAL® (data not shown), corroborating previous results that showed that FhuB and FhuG were also required for iron(III)-hydroxamate uptake (Cabrera et al. (2001) Appl. Environ. Microbiol. 67:1001-3; Sebulsky et al. (2000) J. Bacteriol. 182:4394-4400), but also indicating that an additional iron(III)-siderophore transport system, one that is required for iron-restricted growth in *S*. *aureus,* was interrupted in H1071.

As expected, and in agreement with previous results showing that neither a *fhuB* (Cabrera et al. (2001) Appl. Environ. Microbiol. 67:1001-3) nor *fhuG* (Sebulsky et al. (2000) J. Bacteriol. 182:4394-4400) knockout strain could utilize hydroxamate-type siderophores, the Δ*fhuCBG*::*ermB* mutant was unable to transport iron(III)-hydroxamates as demonstrated by both siderophore plate bioassay and in transport assays, and this defect was fully complemented by the introduction of *fhuCBG in trans.* That the Δ*fhuCBG::ermB* mutant exhibited an iron-dependent growth defect in TMS media alone was surprising, given that strains lacking either *fhuG* or both of *fhuD1* and *fhuD2* do not exhibit any measurable growth defect under similar conditions in comparison to wildtype RN6390. Given our observation that introduction of *fhuC* into H1071 restored the growth defect but did not restore the ability to utilize iron(III)-hydroxamates, suggested that an endogenous iron uptake system was impaired in the mutant.

### Example 10: FhuC is required for iron(III)-staphylobactin transport in S. aureus

FhuC (Sebulsky et al. (2000) J. Bacteriol. 182:4394-4400) shares significant similarity with ATPases and has all the hallmarks (signature sequences) of an ATP-binding protein (Holland and Blight (1999) J. Mol. Biol. 293:381-99). Analysis of the *S*. *aureus* genome identified several operons whose predicted products share significant similarity with iron(III)-siderophore ABC-type transporters and iron(III)-siderophore binding proteins. At least three of these putative operons, SirABC, IsdEF and SA1977-SA1979 (using N315 genome designations), however, lack a gene whose predicted product shares similarity with ATPase components of ABC transporters. Thus, it is possible that FhuC interacts with one or more of these other S. *aureus* ABC transporters to transport an iron-complex that is required for growth under iron limitation. Our previous studies characterized the phenotype of SirA and SirB mutants, both of which demonstrate a growth-impaired phenotype under conditions of iron limitation and which we showed were required for the transport of iron(III)-staphylobactin (Dale et al. (2004) J. Bacteriol. 186:8356-62). Thus, we tested H1071 for its ability to utilize staphylobactin in ⁵⁵Fe-staphylobactin transport assays. These results showed that H1071 could not utilize staphylobactin (Figure 5, inset). Moreover, complementation of this mutant with pFhuCBG and, most notably, pFhuC alone resulted in significant staphylobactin transport (Figure 5), indicating that FhuB and FhuG are not involved in staphylobactin transport and that FhuC (as the ATPase), together with SirABC (SirA, binding protein; SirB and SirC, permease) (Dale et al. (2004) J. Bacteriol. 186:8356-62; Heinrichs et al. (1999) J. Bacteriol. 181:1436-1443) is involved in staphylobactin transport. Although not observed in the 10-minute timeframe of the transport assays, we observed a reproducibly smaller zone of growth in the 48 hour-duration plate bioassays for staphylobactin utilization by H1071 complemented with pFhuCBG compared with complementation with plasmid pFhuC.

We attribute this observation to the possibility that the expression of the FhuB and FhuG proteins in the former might sequester FhuC into a FhuCBG membrane complex, decreasing the possibility for a potential interaction of FhuC with SirABC and, thus, resulting in a decreased ability to transport iron(III)-staphylobactin. However, we cannot rule out the less likely possibility that the decreased staphylobactin utilization in the strain complemented with pFhuCBG is due to poorer expression of FhuC from this construct compared with the strain complemented with pFhuC. The expression of FhuC from both constructs is governed by identical upstream sequences and, as the strains were grown under identical conditions, it is likely that expression of FhuC was fairly equivalent from both constructs.

### Example 11: FhuC interacts with additional iron transporters

We previously showed that a *S*. *aureus sirA* mutant was incapable of transporting staphylobactin (Dale et al. (2004) J. Bacteriol. 186:8356-62), and that this mutant displayed an iron-deficient growth phenotype. However, the lack of *fhuC* in H1071 (although H1071 is deleted for *fhuCBG,* recall that plasmid pFhuC complements the growth defect in this strain) results in a more attenuated growth defect in response to iron starvation than a *sirA* or a *sirB* mutant (Dale et al. (2004) J. Bacteriol. 186:8356-62). Indeed, our use of iron restricted media such as TMS, which contains enough contaminating iron to allow for growth of iron uptake mutants, and the ability to add increasing concentrations of 2,2'-dipyridyl, a non-metabolizable iron chelator, allows us the opportunity to tease apart the contributions of various different iron transporters. Inclusion of 50 µM 2,2'-dipyridyl in TMS strongly attenuates the growth of H1071 compared to wildtype, whereas equivalent growth attenuation is only observed for H306 (RN6390 *sirA*::Km) and H474 (RN6390 *sirB*::Tet) when 2,2'-dipyridyl concentrations in TMS are higher than 100 µM (Dale et al. (2004) J. Bacteriol. 186:8356-62). Together with previously described results, these observations suggest that not only is FhuC interacting with SirABC to transport staphylobactin, but that FhuC is also interacting with an additional transporter that allows the transport of an as yet undetermined iron chelate. In an attempt to determine what additional iron chelates FhuC may participate in the transport of, we utilized plate bioassays with H1071 using a range of potential iron sources, including enterobactin, ferric citrate, hemin, and hemoglobin. Under these conditions, H1071 was not significantly impaired in the utilization of any of these additional iron sources (data not shown).

Based on these results it is apparent that *fhuC* is required for the transport of iron(III)-hydroxamate siderophores and the as yet structurally uncharacterized staphylobactin siderophore. The data also support the conclusion that *fhuC* is likely involved in the transport of an additional iron acquisition system (i.e., in addition to *fhu* and sir-encoded systems). This is based on the observation that the iron-restricted growth defect displayed by the Δ*fhuCBG* mutant is more severe than that of either a *sirA* or *sirB* mutant during growth in low-iron conditions. At present the identity of the additional pathway(s) affected by the deletion of *fhuC* is unknown, however, the *fhuCBG* mutant was still able to utilize hemin, hemoglobin, ferric citrate, and the catechole siderophore enterobactin at levels equivalent to RN6390 in bioassays, demonstrating that the loss of *fhuC* does not affect the ability of *S. aureus* to obtain iron from these sources. It is tempting to speculate that FhuC interacts with one or both of IsdEF or SA1977-1979 to provide the ATPase component that is genetically unlinked to these transporters. Proteins encoded by the *isd* locus have been implicated in interactions with host iron complexes (Mack et al. (2004) Biochem. Biophys. Res. Commun. 320:781-8; Mazmanian et al. (2003) Science 299:906-9; Skaar and Schneewind. (2004) Microbes Infect. 6:390-7), complexes that were not present in the TMS laboratory media that was used to demonstrate the iron-restricted growth defect. It was therefore not surprising to find that H1071 was not impaired, compared to wildtype, in heme or hemoglobin uptake. The genetic region surrounding SA1977-1979 contains several open reading frames whose putative products share similarity with siderophore biosynthetic enzymes and it is therefore possible that the lack of FhuC in H1071 abrogates the utilization of another staphylococcal siderophore whose production requires the expression of genes within this locus.

### Example 12: The ΔfhuCBG::ermB mutant displays an altered pathogenicity profile in a mouse kidney abscess model

We have previously shown that siderophore biosynthesis is important for *S*. *aureus* virulence in a mouse kidney abscess model (Dale et al. (2004) Infect. Immun. 72:29-37). Since we showed that H1071 (RN6390 *ΔfhuCBG*::*ermB*) was compromised for staphylobactin uptake, we were interested in determining if this mutant was also attenuated in this model system. Groups of seven Swiss-Webster mice were inoculated with 10⁷ CFU of *S*. *aureus* Newman or H1074 (Newman *ΔfhuCBG*::*ermB*) via the tail vein and the mice were monitored daily for grooming and locomotory function. Each day, the mice were assigned a subjective score that reflected their relative health. For example, mice that received a score of 1 were normal for both grooming and locomotion, whereas mice that received scores of 4 were completely moribund. To account for death prior to the endpoint of the experiment (one mouse infected with Newman died on day 6), we divided clinical scores by the number of days surviving to arrive at a final clinical score. Mice that were injected with H1074 were significantly less moribund than mice injected with *S*. *aureus* Newman (Table 5; clinical scores of 1.8 vs. 2.7, respectively - a completely healthy mouse would have a clinical score of 1.4.) On day 7, mice were sacrificed and kidneys were removed and evaluated for abscess formation. Eight of fourteen kidneys (57%) removed from mice that were injected with *S*. *aureus* Newman were abscessed, whereas only 2 of 14 kidneys (14%) removed from mice injected with H1074 showed visible abscess formation. These results were shown to be highly significant (p < 0.0001) using the Z-test and Fisher's Exact test. Interestingly, however, when kidney homogenates were plated for enumeration of total bacteria, no significant differences in bacterial load in the kidneys between mice injected with Newman versus H1074 (data not shown).

These results demonstrated that the pathogenicity of a Newman Δ*fhuCBG*::*ermB* strain was altered in comparison to the Newman parent strain since, compared to wildtype, the mutant H1074 caused significantly less kidney abscesses and less weight loss without a reduction in bacterial load in the kidneys. These results indicated that the lack of the potential to express FhuCBG (in vivo expression of the *fhu* genes has not been thus far demonstrated but is inferred based upon their iron-regulated expression profile in vitro) does not affect the ability of the bacteria to persist in vivo, but does seem to lessen their potential to cause more severe infection. These results contrast with previous results showing that a Newman *sbnE::Km* mutant (unable to produce staphylobactin siderophore) did result in less bacteria in kidneys at 6 days post infection. The fact that expression of FhuCBG, at least in this model system, appears to be required for the full virulence potential of *S. aureus* is in agreement with the conservation of this operon within all *S*. *aureus* genomes sequenced to date (Sebulsky et al. (2004) J. Biol. Chem. 279:53152-9), in contrast to *fhuD1* which is absent in the genomes of N315 and MRSA252.

### Example 13: Interaction Assays

Assays to screen for agents that disrupt the interaction of Sir polypeptides, staphylobactin, and/or FhuC protein will be conducted as follows. A 96-well microplate with high protein adsorption capacity will be coated with streptavidin overnight at 4°C at a concentration of 30 µg/ml in 1 times PBS buffer (0.15 M NaCl, pH 6.8). After removal of the solution, the plate will be blocked with 2% BSA in PBS buffer for one or more hours at room temperature. The plate will then be washed and used in the assay. For example, biotinylated SirA and SirB in the absence or presence of a test agent will be incubated for 45 to 60 minutes at room temperature. After three washes with water, an anti-SirB antibody mixed with a secondary antibody conjugated to either alkaline phosphatase (AP) or horseradish peroxidase (HRP) will be added and incubated for one hour. The plate will then be washed to separate the bound from the free antibody complex. A chemiluminescent substrate (alkaline phosphatase or Super Signal luminol solution from Pierce for horseradish peroxidase) will be used to detect bound antibody. A microplate luminometer will be used to detect the chemiluminescent signal. The absence of the signal will indicate that the test agent inhibits or disrupts the binding of sirA to sirB. Similar assays may also be conducted to identify agents that disrupt the interaction of sirA and sirC, sirB and sirC, and/or sirA, sirB, sirC, staphylobactin and/or FhuC.

### Example 14: Iron-transport Assays

Assays to screen for agents that disrupt the iron transport system of *S*. *aureus* will be conducted as follows. Wild type *S*. *aureus* cells and SirA deficient *S. aureus* cells will be cultured in tryptic soy broth (TSB) (Difco). Cells will be washed before approximately 10⁷ colony forming units of each strain are inoculated into fresh TMS media containing with 50 µM FeCl₃ in the presence or absence of a test agent. Bacterial growth will be monitored using a Klett meter until late stationary phase was reached.

A test agent that alters the growth rate of wild type *S*. *aureus* cells but not SirA deficient cells is likely toxic to cells in the presence of intracellular iron and, therefore, cells importing iron are generally more sensitive to the toxic affects of this drug versus mutants debilitated in iron import.

Similar transport assays may be conducted for SirB, SirC, and FhuC using SirB, SirC, and/or FhuC-deficient cells.

### Example 15: Expression Assays

Assays to screen for agents that disrupt the expression of SirA in *S. aureus* will be conducted as follows. Wild type *S*. *aureus* cells will be cultured overnight in tryptic soy broth (TSB) (Difco) in the presence or absence of a test agent. Following 24 hours of culture, the cells will be washed in IX PBS (phosphate buffered saline) and then lysed at 37°C using 10 µg of lysostaphin in STE (0.1 M NaCl, 10 mM Tris-HCl [pH 8.0], 1 mM EDTA [pH 8.0]). The cell lysates will then be transferred to anti-SirA antibody precoated plates and incubated for 45 to 60 minutes at room temperature. As a control, cell lysates from untreated *S. aureus* cells will be used. After three washes with water, a secondary antibody conjugated to either alkaline phosphatase (AP) or horseradish peroxidase (HRP) will be added and incubated for one hour. The plate will then be washed to separate the bound from the free antibody complex. A chemiluminescent substrate (alkaline phosphatase or Super Signal luminol solution from Pierce for horseradish peroxidase) will be used to detect bound antibody. A microplate luminometer will be used to detect the chemiluminescent signal. The absence of the signal in samples of cell lysates obtained from cells treated with test agent will indicate that the test agent inhibits the expression of SirA. Similar expression assays may also be conducted for SirB, SirC, staphylobactin, and/or FhuC.

The practice of the present invention will employ, unless otherwise indicated, conventional techniques of cell biology, cell culture, molecular biology, transgenic biology, microbiology, recombinant DNA, and immunology, which are within the skill of the art. Such techniques are described in the literature. See, for example, Molecular Cloning: A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Antibodies: A Laboratory Manual, and Animal Cell Culture (R. I. Freshney, ed. (1987)), Manipulating the Mouse Embryo, (Cold Spring Harbor Laboratory Press, Cold Spring Harbor, N.Y., 1986).

**Table 1. Bacterial strains and plasmids used in Examples 2-5**

| Bacterial strains, plasmids or oligonucleotides | Description^{a} | Source or reference |
|---|---|---|
| ***E. coli*** | | |
| DH5α | *φ80dlac*ZΔM15 *recAl endAl gyrA96 thi-1 hsdr17*(r_{κ}*⁻* m_{κ}⁺) *supE44 relAl deoR Δ(lacZYA-argF)*U169 | Promega |
| ER2566 | *F⁻λ⁻ fhuA2 [lon] ompT* /*acZ::T7 geneI gal sulAll Δ (mcrC-mrr)114::IS10 R(mcr-73::miniTnl0)2 R(zgb-210::Tnl0)1* (Tet^{s}) *endA1 [dcm]* | New England Biolabs |
| | | |
| ***S. aureus*** | | |
| RN4220 | Restriction-deficient; accepts foreign DNA | Lab stock |
| 8325-4 | Prophage-cured wild-type strain | Lab stock |
| RN6390 | Prophage-cured wild-type strain | Lab stock |
| Newman | Clinical isolate; wild-type strain | Lab stock |
| H287 | RN6390 *fhuG*::Tn917; Em^{r} | Sebulsky¹ |
| H306 | RN6390 *sirA*::Km; Km^{r} | This study |
| H474 | RN6390 *sirB*::Tet; Tet^{r} | This study |
| H686 | Newman *sbnE::Km* | Dale² |
| H706 | Newman *fur*::Km; Km^{r} | This study |
| H789 | RN6390 *sirA*::Km, *sbnF*::pMUTIN4; Km^{r} Em^{r} | This study |
| H790 | RN6390 *sirA*::Km, *sbnI*::pMUTIN4; Km^{r}Em^{r} | This study |
| H791 | RN6390 *sirA*::Km, *sbnA*::pMUTIN4; Km^{r}Em^{r} | This study |
| H796 | RN6390 *sirB*::Tet *sbnF:*:pMUTIN4; Tet^{r} Em^{r} | This study |
| H797 | RN6390 *sirB*::Tet *sbnI:*:pMUTIN4; Tet^{r} Em^{r} | This study |
| H800 | RN6390 *sirB*::Tet *sbnA*::pMUTIN4; Tet^{r} Em^{r} | This study |
| H803 | Newman *sirA*::Km: Km^{r} | This study |
| H804 | Newman *sirB*::Tet; Tet^{r} | This study |
| H870 | Newman *sbnH*::pMUTIN4 | This study |
| H873 | H803 *sbnH*::pMUTIN4 | This study |
| H876 | H804 *sbnH*::pMUTIN4 | This study |
| | | |
| ***Plasmids*** | | |
| pGEX-2T-TEV | Expression vector for generating protein fusions with GST | Sebulsky³ |
| pALC2073 | that are cleavable with tobacco etch virus protease *E. coli* - *S*. *aureus* shuttle vector, contains P_{xyl/tet}; Cm^{r} | A. Cheung |
| pAUL-A | Temperature sensitive *E. coli* - *S. aureus* shuttle vector; | Chakraborty⁴ |
| pAW8 | *E. coli* - *S. aureus* shuttle vector; Tef | A. Wada |
| pBC SK+ | E. coli phagemid; Cm^{r} | Stratagene |
| pDG782 | pMLT22 derivative that carries a kanamycin resistance cassette; Ap^{r} Km^{r} | Guerout-Fleury⁵ |
| pDG1513 | pMLT22 derivative that carries a tetracycline resistance cassette; Cm^{r} Te^{r} | Guerout-Fleury⁵ |
| pMTS 12 | pAUL-A derivative carrying *sirA*::Km; Km^{r} Em^{r} | This study |
| pMUTIN4 | lacZ fusion vector; Ap^{r} (*E*. *coli*) Em^{r} (*S. aureus*) | Vaguer⁶ |
| pSED43 | pALC2073 derivative carrying the *sirB* coding region; Cm^{r} | This study |
| pSED44 | pAW8 derivative carrying *sirABC*; Tet^{r} | This study |
| pSirA | pGEX-2T-TEV derivative carrying *sirA*; Amp^{r} | This study |
| pSirABC | pBC SK+ carrying *sirABC*; Cm^{r} | This study |
| pSirB::Tet3 | pAUL-A derivative carrying *sirB*::tet; Tet^{r} Em^{f} | This study |
| | | |
| Oligonucleotides^{b} | | |
| pSirA (BamHI) | GCAATGGGTACAGGATCCATTAAAGGGAAACCAAAG | |
| pSirA (EcoRI) | TTGAATTCGTAGCATCGTAAAACTCCTT | |
| SirB Comp 5' | TTGGTACCGGCGGATATAAATCTTCATT | |
| SirB Comp 3' | TTGAGCTCTTTCGGTCATAAGCGTTGAC | |
| Sir Upper | TCACGAAGGAGGCTAATTAG | |
| Sir Lower | CCTCGCAACGGTTAGTTAAC | |
| SirB Internal 5' | 5' CAGCTACGGCTACCGAAATA | |
| SirB Internal 3' | 5' CATTTTTGGGGGCTATTGTTGT | |
| Gapdh 5' | GGAGGCCATTACCATGGCAG | |
| Gapdh 3' | TGCTCCCCGCTTACTCATAA | |

| | | |
|---|---|---|
| ^{a} Abbreviations: Cm^{r}, Tet^{r}, Em^{r}, Km^{r}, Amp^{r}: resistance to chloramphenicol, tetracycline, erythromycin, kanamycin and ampicillin, respectively. ^{b} Restriction endonuclease recognition sites are underlined. ¹Sebulsky et al. (2000) J. Bacteriol. 182:4394-4400 ²Dale et al. (2004) Infect. Immun. 72:29-37 ³Sebulsky et al. (2003) J. Biol. Chem. 278:49890-900. ⁴Chakraborty et al. (1992) J. Bacteriol. 174:568-574 ⁵Guerout-Fleury et al. (1995) Gene 167:335-336 ⁶Vaguer et al. (1998) Microbiology 144:3097-3104 | | |

**TABLE 2. Minimum inhibitory concentrations (MIC) of streptonigrin and 2,2'-dipyridyl against S. aureus Newman and derivatives**

| | MIC^{a} | |
|---|---|---|
| Bacterial strain | Streptonigrin (ng/ml) | 2,2'-dipyridyl (µM) |
| Newman | 2 | 500 |
| H803 | 8 | 125 |
| H804 | 8 | 125 |
| Newman + 50 µM DESFERAL® | 2 | nd*^{b}* |
| H803 + 50 µM DESFERAL® | 2 | nd |
| H804 + 50 µM DESFERAL® | 2 | nd |

| | | |
|---|---|---|
| ^{a} **bacteria were grown in TMS** *^{b}* **not determined** | | |

**TABLE 3. β-galactosidase activity from sbn-lacZ fusions in Newman and derivatives grown in iron-restricted media**

| Values represent the mean values, in triplicate, from assays performed on triplicate cultures | |
|---|---|
| Bacterial Strain | Mean (β-galactosidase activity ± SD (RLU/s) |
| Newman | 172 ± 117 |
| H803 | 318 ± 47 |
| H804 | 239 ± 110 |
| H870: Newman *sbnH::pMUTIN4* | 825 ± 190 |
| H873: H803 sbnH::pMUTIN4 | 7036 ± 517 |
| H876: H804 sbnH::pMUTIN4 | 3667 ±1654 |

**Table 4. Bacterial strains and plasmids used in Examples 7-12**

| Strain or plasmid | Description*^{a}* | Source or reference |
|---|---|---|
| Strains | | |
| *S. aureus* | | |
| | | |
| RN4220 | r_{K}⁻ m_{K}⁺; capable of accepting foreign DNA | Kreiswirth¹ |
| RN6390 | Prophage cured wild-type strain | Peng² |
| Newman | Wild-type strain | Duthie³ |
| H287 | RN6390 *fhuG*::Tn*917*; Em^{r} | Sebulsky⁴ |
| H431 | *RN6390 fhuD1*::Km *fhuD2::Tet*; Km^{r} Tet^{r} | Sebulsky⁵ |
| H1068 | RN4220 *ΔfhuCBG*::*ermB*; Em^{r} | This study |
| H1071 | RN6390 Δ*fhuCBG*::*ermB*; Em^{r} | This study |
| H1074 | Newman Δ*fhuCBG*::*ermB*; Em^{r} | This study |
| *E. coli* | | |
| DH5α | φ80d*lacZ*ΔM15 *recAl endAl gyrA96 thi*-1 *hsdR17*(rₖ⁻ mₖ⁺) *supE44 relAl deoR Δ*(*lacZYA-argF*)U169 | Promega |
| | | |
| Plasmids | | |
| pAUL-A | Temperature sensitive *S. aureus* suicide vector; Em^{r} | Chakraborty⁶ |
| pAUL-A Km | pAUL-A containing the 1.6-kb kanamycin resistance cassette (inserted as a *Cla*I fragment) from pDG782; Em^{r} Km^{r} | This study |
| pDG646 | pSB119 derivative carrying an erythromycin resistance cassette; Ap^{r} Em^{r} | Guerout-Fluery⁷ |
| pDG782 | pMTL22 derivative carrying a kanamycin resistance cassette; Ap^{r} Km^{r} | Guerout-Fluery⁷ |
| pΔFhuCBG | pAULA Km derivative carrying *fhuCBG*::*ermB*; Em^{r} Km^{r} | This study |
| pLI50 | 5.2-kb *E.coli*/*S. aureus* shuttle vector; Ap^{r} Cm^{r} | Lee⁸ |
| pUC19 | General purpose *E. coli* cloning vector; Ap^{r} | Sambrook⁹ |
| pFhuC | pLI50 containing the *S*. *aureus fhuC* gene; Ap^{r} Cm^{r} | This study |
| pFhuCBG | pLI50 containing the *S*. ***aureus** fhuCBG* operon; Ap^{r} Cm^{r} | This study |

| | | |
|---|---|---|
| *^{a}* Abbreviations: Ap^{r}, Cm^{r}, Em^{r}, Km^{r}, Tet^{r} designate resistance to ampicillin, chloramphenicol, erythromycin, kanamycin, and tetracycline, respectively. ¹Kreiswirth et al. (1983) Nature 305:680-685. ²Peng et al. (1988) J. Bacteriol. 170:4365-4372. ³Duthie and Lorenz (1952) J. Gen. Microbiol. 6:95-107. ⁴Sebuskly et al. (2000) J. Bacteriol. 182:4394-4400. ⁵Sebuskly and Heinrichs (2001) J. Bacteriol. 183:4994-5000. ⁶Chakraborty et al. (1992) J. Bacteriol. 174:568-574. ⁷Guerout-Fleury et al. (1995) Gene 167:335-336. ⁸Lee (1992) Mol. Microbiol. 6:1515-1522. ⁹Sambrook et al. (1989) Molecular Cloning. A laboratory manual, 2nd ed. Cold Spring Harbor Laboratory Press, Cold Spring Harbor. | | |

**Table 5. Clinical characteristics of mice infected with S. aureus Newman and H1074.**

| **Mouse Number and Infecting Strain** | **Abscess Formation^{a}** | **% Weight Loss (Gain)** | **Clinical Score^{b}** |
|---|---|---|---|
| 1-Newman | A/A | 26 | 3.3 |
| 2-Newman | A/A | 33 | 2.9 |
| 3-Newman | A/- | 31 | 2.6 |
| 4-Newman | -/- | 13 | 2.9 |
| 5-Newman^{c} | A/A | 43 | 3.8 |
| 6-Newman | -/- | 7 | 1.6 |
| 7-Newman | A/- | 12 | 2.0 |
| **Averages:^{d}** | | 22.1 ± 4.8 | 2.7 ± 0.3 |
| | | | |
| 1-H1074 | -/- | 8 | 1.4 |
| 2-H1074 | -/- | 19 | 1.7 |
| 3-H1074 | A/A | 33 | 2.7 |
| 4-H1074 | -/- | 26 | 2.4 |
| 5-H1074 | -/- | 15 | 1.9 |
| 6-H1074 | -/- | 4 | 1.4 |
| 7-H1074 | -/- | (1) | 1.4 |
| **Averages:** | | 14.8 ± 4.6 | 1.8 ± 0.2 |
| | | | |
| 1-Saline Injection | -/- | (12) | 1.4 |

| | | | |
|---|---|---|---|
| ^{a} A/A, both kidneys visibly abscessed; A/-, one kidney visibly abscessed; -/- neither kidney visibly abscessed. ^{b} Clinical scores were based on grooming and locomotory ability over the course of the experiment, normalized to the number of days the mice survived. ^{c} Mouse was sacrificed on Day 6 due to extreme morbidity. ^{d} Averages are ± standard error of the mean. | | | |

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method for identifying an agent that inhibits an interaction between *Staphylococcus aureus (S. aureus)* proteins selected from the group consisting of SirA and SirB, SirA and SirC, SirB and SirC, SirB and FhuC and SirC and FhuC comprising:
(i) contacting the proteins, or nucleic acid encoding the proteins, in the presence and absence of an agent under conditions permitting the interaction between the two proteins; and
(ii) determining the level of interaction between the proteins, wherein a different level of interaction between the proteins in the presence of the agent relative to in the absence of the agent indicates that the agent inhibits the interaction between the proteins.

2. The method of claim 1, wherein the agent is a small organic molecule, a peptide, a polypeptide, a peptide mimetic or an antibody.

3. The method of claim 1, wherein the agent is a polynucleotide.

4. The method of claim 3, wherein the agent is an antisense polynucleotide.

5. The method of claim 3, wherein the agent is siRNA.

6. The method of any preceding claim, wherein the agent inhibits the interaction between FhuC and SirB or SirC.

7. Use of an agent to inhibit growth of *S. aureus* cells, wherein the agent specifically hybridizes or binds with mRNA or genomic DNA encoding a polypeptide having an amino acid sequence of SEQ ID NO: 16.

## Patentansprüche

1. Verfahren zum Identifizieren eines Agens, das eine Wechselwirkung zwischen *Staphylococcus aureus* (*S. aureus*)-Proteinen, ausgewählt aus der Gruppe bestehend aus SirA und SirB, SirA und SirC, SirB und SirC, SirB und FhuC und SirC und FhuC, hemmt, wobei das Verfahren Folgendes umfasst:
(i) Inkontaktbringen der Proteine oder der Nukleinsäuren, die für die Proteine codieren, in Gegenwart bzw. Abwesenheit eines Agens unter Bedingungen, die die Wechselwirkung zwischen den beiden Proteinen gestatten; und
(ii) Bestimmen des Ausmaßes der Wechselwirkung zwischen den Proteinen, wobei ein unterschiedliches Ausmaß an Wechselwirkung zwischen den Proteinen in Gegenwart des Agens im Vergleich zu in Abwesenheit des Agens anzeigt, dass das Agens die Wechselwirkung zwischen den Proteinen hemmt.

2. Verfahren nach Anspruch 1, wobei es sich bei dem Agens um ein kleines organisches Molekül, ein Peptid, ein Polypeptid, ein Peptidmimetikum oder einen Antikörper handelt.

3. Verfahren nach Anspruch 1, wobei es sich bei dem Agens um ein Polynukleotid handelt.

4. Verfahren nach Anspruch 3, wobei es sich bei dem Agens um ein Antisense-Polynukleotid handelt.

5. Verfahren nach Anspruch 3, wobei es sich bei dem Agens um siRNA handelt.

6. Verfahren nach einem vorhergehenden Anspruch, wobei das Agens die Wechselwirkung zwischen FhuC und SirB oder SirC hemmt.

7. Verwendung eines Agens zum Hemmen des Wachstums von *S*. *aureus*-Zellen, wobei das Agens spezifisch an mRNA oder genomische DNA, die für ein Polypeptid mit einer Aminosäuresequenz gemäß SEQ ID NO: 16 codiert, hybridisiert oder bindet.

## Revendications

1. Procédé d'identification d'un agent qui inhibe l'interaction entre des protéines de *Staphylococcus aureus* (*S. aureus*) choisies dans le groupe constitué de SirA et SirB, SirA et SirC, SirB et SirC, SirB et FhuC et SirC et FhuC, comprenant :
(i) la mise en contact des protéines, ou d'acides nucléiques codant pour les protéines, en présence et en l'absence d'un agent dans des conditions permettant l'interaction entre les deux protéines ; et
(ii) la détermination du niveau d'interaction entre les protéines, dans lequel un niveau d'interaction différent entre les protéines en présence de l'agent par rapport à en l'absence de l'agent indique que l'agent inhibe l'interaction entre les protéines.

2. Procédé selon la revendication 1, dans lequel l'agent est une petite molécule organique, un peptide, un polypeptide, un mimétique de peptide ou un anticorps.

3. Procédé selon la revendication 1, dans lequel l'agent est un polynucléotide.

4. Procédé selon la revendication 3, dans lequel l'agent est un polynucléotide antisens.

5. Procédé selon la revendication 3, dans lequel l'agent est un petit ARN interférent.

6. Procédé selon l'une quelconque des revendications précédentes, dans lequel l'agent inhibe l'interaction entre FhuC et SirB ou SirC.

7. Utilisation d'un agent pour inhiber la croissance de cellules de *S*. *aureus,* dans laquelle l'agent s'hybride ou se lie de façon spécifique avec un ARNm ou un ADN génomique codant pour un polypeptide présentant la séquence d'acides aminés SEQ ID NO : 16.
